# EUROPEAN PATENT SPECIFICATION

(11) **EP 3 082 857 B1**
(45) Date of publication and mention of the grant of the patent: **25.07.2018**
(21) Application number: 14821592.4
(22) Date of filing: 17.12.2014
(51) Int. Cl.: A61K 39/395, C07K 16/22, C07K 16/28, A61K 39/00

(54) **COMBINATION THERAPY WITH AN ANTI-ANG2 ANTIBODY AND A CD40 AGONIST**
KOMBINATIONSTHERAPIE MIT EINEM ANTI-ANG2 ANTIKÖRPER UND EINEM CD40-AGONISTEN
THÉRAPIE DE COMBINAISON AVEC UN ANTICORPS ANTI-ANG2 ET UN AGONISTE DE CD40

(30) Priority: 20.12.2013 EP 13198753; 07.03.2014 EP 14158331
(43) Date of publication of application: 26.10.2016
(73) Proprietor: F. Hoffmann-La Roche AG, 4070 Basel (CH)
(72) Inventor: KLEIN, Christian, CH-8906 Bonstetten (CH); MUELLER, Philipp, 88400 Biberach an der Riss (DE); THOMAS, Markus, 79618 Rheinfelden (DE); ZIPPELIUS, Alfred, CH-4059 Basel (CH)
(74) Representative: Burger, Alexander
(86) International application number: PCT/EP2014/078233
(87) International publication number: WO 2015/091655

(56) References cited:
- WO-A1-2013/132044
- STEPHEN J DEMAREST ET AL: "Emerging antibody combinations in oncology", MABS, LANDES BIOSCIENCE, US, vol. 3, no. 4, 1 July 2011 (2011-07-01), pages 338-351, XP002690158, ISSN: 1942-0870, DOI: 10.4161/MABS.3.4.16615
- Q. JIANG ET AL: "mTOR Kinase Inhibitor AZD8055 Enhances the Immunotherapeutic Activity of an Agonist CD40 Antibody in Cancer Treatment", CANCER RESEARCH, vol. 71, no. 12, 3 May 2011 (2011-05-03), pages 4074-4084, XP055099556, ISSN: 0008-5472, DOI: 10.1158/0008-5472.CAN-10-3968
- T. S. LEWIS ET AL: "Distinct Apoptotic Signaling Characteristics of the Anti-CD40 Monoclonal Antibody Dacetuzumab and Rituximab Produce Enhanced Antitumor Activity in Non-Hodgkin Lymphoma", CLINICAL CANCER RESEARCH, vol. 17, no. 14, 15 July 2011 (2011-07-15) , pages 4672-4681, XP055059121, ISSN: 1078-0432, DOI: 10.1158/1078-0432.CCR-11-0479

## Description

The present invention relates to the combination therapy of an antibody that binds to human angiopoietin 2 (ANG-2) with a CD40 agonist.

### Background of the Invention

Angiopoietins, which play a key role in angiogenesis and blood vessel remodeling, are part of the pro-angiogenic armamentarium of growing tumors. Importantly they are one of the major factors leading to secondary resistance during anti-VEGF therapy (Saharinen, P., et al., Trends Mol Med 17 (2011) 347-362). Both angiopoietin-1 (Ang1) and angiopoietin-2 (Ang2) are Tie2-receptor ligands. While Ang1 tends to stabilize and matures blood vessel (Yancopoulos, G. D., et al., Nature 407 (2000) 242-248) Ang2 promotes tumor angiogenesis and growth by destabilizing blood vessels. Ang2 thereby opposes Ang1 in its function (Cascone, T. et al, J Clin Oncol 30 (2012) 441-444). Along this line it has been observed that blocking Ang2, but not Ang1 normalizes tumor blood vessels (Falcon, B. L., H. Hashizume, et al., Am J Pathol 175 (2009) 2159-2170) and helps to overcome acquired resistance towards anti-VEGF therapy (Chae, S. S., W. S. Kamoun, et al., Clin Cancer Res 16 (2010) 3618-3627; Thomas, M., et al. PLoS One 8 (2013) e54923).

Immunomodulatory antibodies offer an treatment approach and might be used to directly potentiate anti-tumor immune responses or as adjuvants for anti-cancer vaccines (Melero, I., et al. Nat Rev Cancer 7 (2007) 95-106). Agonistic anti-CD40 antibodies constitute one of the most effective classes of these reagents. CD40 is a cell-surface member of the tumor necrosis factor superfamily expressed on antigen presenting cells (APCs) such as dendritic cells, B cells and macrophages. Preclinical studies with anti-CD40 agonists suggest that triggering CD40 with crosslinking antibodies on antigen presenting cells (APCs) can substitute for CD4 T cell help, normally provided via CD40 ligand, and facilitate the activation as well as expansion of CD8 effector T cells (Li, F. et al, Science 333 (2011) 1030-1034). In addition CD40-activated macrophages may also exert direct tumoricidal functions (Beatty, G. L., et al. Science 331 (2011) 1612-1616); Vonderheide, R. H., et al. Oncoimmunology 2 (2013) e23033).

The immuno-suppressive tumor microenvironment poses a major hurdle for anti-tumor immunity and immunotherapeutic treatment approaches (Beatty, G. L., et al. Science 331 (2011) 1612-1616).

WO 2013/132044 A1 (HOFFMANN LA ROCHE [CH]; CANNARILE MICHAEL [DE]; RIES CAROLA [DE]; RUET) discloses methods of treatment of cancer using anti-CSF-1R antibodies in combination with either anti-ANG-2 antibodies or anti-CD40 agonistic antibodies. STEPHEN J DEMAREST ET AL, 2011 ("Emerging antibody combinations in oncology", MABS, vol. 3, no. 4, pages 338-351) discloses that anti-ANG-2 antibodies and anti-CD40 agonistic antibodies are known as anti-cancer agents.

### Summary of the Invention

The present inventors have found that anti-ANG2 antibodies enhance the efficacy of CD40 agonists to treat cancers or delay progression of a tumor or the survival of a patient afflicted with cancer e.g. with a solid tumor. The delay of progression, the longer survival as well as the potential of reduced doses with lower risk of side effects represent a major benefit for patients.

Surprisingly a treatment of tumors with anti-ANG2 antibodies in combination with CD40 agonists showed strong synergistic effect on the time to tumor progression and the time of survival (whereas a combination of anti-VEGF antibodies in combination with CD40 agonists only showed small effects).

One aspect of the invention is an antibody that binds to human angiopoietin 2 (ANG-2)
a) for use in a method of treating or delaying progression of cancer, or
b) for use in a method of prolonging the survival of a patient suffering from cancer, wherein the method comprises administration of said antibody in combination with an agonistic CD40 antibody.

In one embodiment said anti-ANG2 antibody is for use in stimulating an immune response or function, such as T cell activity, , preferably CD8 effector T cell activity, or macrophage activity, preferably CD40-activated macrophage activity. In one embodiment said anti-ANG2 antibody is for use in rendering a cancer susceptible for the treatment with an antibody that binds to human angiopoietin 2 (ANG-2).

Another aspect of the invention is an agonistic CD40 antibody a) for use in a method of treating or delaying progression of cancer, or b) for use in a method of prolonging the survival of a patient suffering from cancer, wherein the method comprises administration of said antibody in combination with an anti-ANG2 antibody.

In one embodiment said agonistic CD40 antibody is for use in stimulating an immune response or function, such as T cell activity, preferably CD8 effector T cell activity, or macrophage activity, preferably CD40-activated macrophage activity.

In one embodiment said agonistic CD40 antibody is for use in rendering a cancer susceptible for the treatment with an antibody that binds to human angiopoietin 2 (ANG-2).

In one embodiment, the anti-ANG2 antibody is a human or humanized antibody.

Preferably, the anti-ANG2 antibody specifically binds to human ANG2 with a K_{D} value of less than 1.0 x 10⁻⁸ mol/l, as determined by surface plasmon resonance (Biacore™).

The anti-ANG2 antibody is preferably an IgG antibody and more preferably, the anti-ANG2 antibody is of human IgG1 or IgG4 subclass.

In one preferred embodiment the anti-ANG2 antibody inhibits the interaction of human ANG-2 with TIE2 receptor with an IC50 of 15 nM or less.

In one embodiment,
i) the anti-ANG2 antibody comprises
   (a) a heavy chain variable domain amino acid sequence of SEQ ID NO:1 and a light chain variable domain amino acid sequence of SEQ ID NO:2; or
   (b) a heavy chain variable domain amino acid sequence of SEQ ID NO:3 and a light chain variable domain amino acid sequence of SEQ ID NO:4;
   and
ii) the agonistic CD40 antibody comprises
   (a) a heavy chain variable domain amino acid sequence of SEQ ID NO: 5 and a light chain variable domain amino acid sequence of SEQ ID NO: 6; or
   (b) a heavy chain variable domain amino acid sequence of SEQ ID NO: 7 and a light chain variable domain amino acid sequence of SEQ ID NO: 8;

In one preferred embodiment
i) the anti-ANG2 antibody comprises
   a heavy chain variable domain amino acid sequence of SEQ ID NO:1 and a light chain variable domain amino acid sequence of SEQ ID NO:2; or
   and
ii) the agonistic CD40 antibody comprises
a heavy chain variable domain amino acid sequence of SEQ ID NO: 5 and a light chain variable domain amino acid sequence of SEQ ID NO: 6; or

In one preferred embodiment the anti-ANG2 antibody is a bispecific antibody that binds to human ANG-2 and that binds to human VEGF.

In one preferred embodiment,
i) the bispecific antibody that binds to human ANG-2 and that binds to human VEGF comprises
   a heavy chain variable domain amino acid sequence of SEQ ID NO:1 and a light chain variable domain amino acid sequence of SEQ ID NO:2; and
   a heavy chain variable domain amino acid sequence of SEQ ID NO:9 and a light chain variable domain amino acid sequence of SEQ ID NO:10;
   and
ii) the agonistic CD40 antibody comprises
a heavy chain variable domain amino acid sequence of SEQ ID NO: 5 and a light chain variable domain amino acid sequence of SEQ ID NO: 6.

In one preferred embodiment
i) the bispecific antibody that binds to human ANG-2 and that binds to human VEGF comprises the amino acid sequences of SEQ ID NO: 11, of SEQ ID NO: 12, of SEQ ID NO: 13, and of SEQ ID NO: 14; and
ii) the agonistic CD40 antibody comprises
a heavy chain variable domain amino acid sequence of SEQ ID NO: 5 and a light chain variable domain amino acid sequence of SEQ ID NO: 6.

In one preferred embodiment the cancer comprises a solid tumor.

In one preferred embodiment the cancer is colorectal cancer, ovarian cancer, glioblastoma, gastric cancer, pancreatic cancer, breast cancer, lung cancer, hepatocellular cancer.

### Description of the Figures

- **Figure 1**: In vivo anti-tumor efficacy of Anti-ANG2 antibodies (and Anti-ANG2 + anti-VEGF antibodies (=Anti-VEGF/ANG2)) in combination with an agonistic CD40 antibody (delaying of progression (tumor growth) of cancer and prolonging the survival of patients treated) - The graph represents pooled data from 3 independent experiments. Mice were treated with the antibody combinations indicated in the figure.

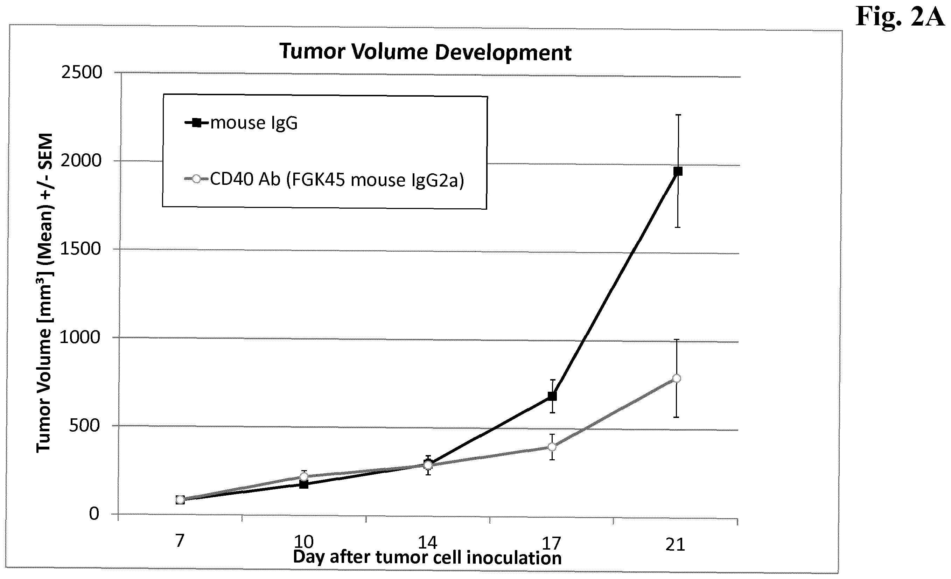

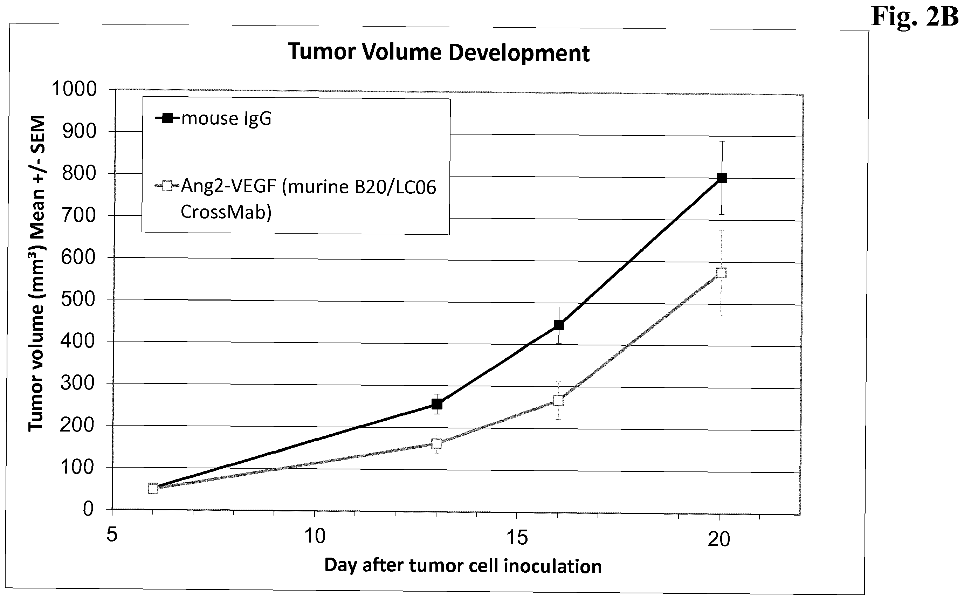

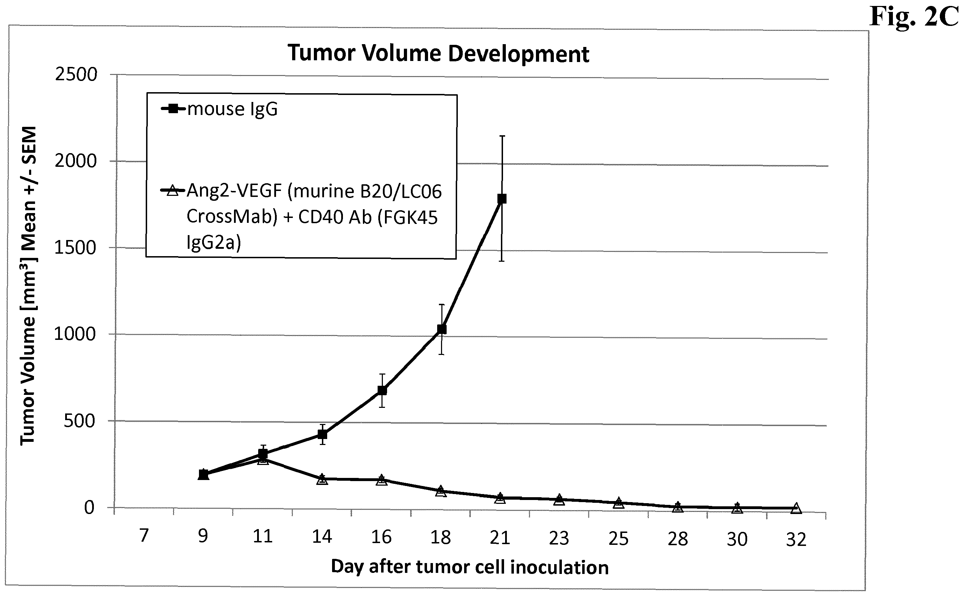

- **Figure 2**: In vivo anti-tumor efficacy of Anti-ANG2 antibodies (bispecific) in combination with an agonistic CD40 antibody in subcutaneous syngeneic MC38 colon carcinoma model.
**2A:** Tumor growth inhibition of agonistic CD40 antibody monotheraphy (unfilled circles) compared to control (filled squares).
**2B:** Tumor growth inhibition of bispecific ANG2/VEGF antibody monotheraphy (unfilled squares) compared to control (filled squares).
**2C:** Tumor growth inhibition of the combination of agonistic CD40 antibody and bispecific ANG2/VEGF antibody (unfilled triangles) compared to control (filled squares).

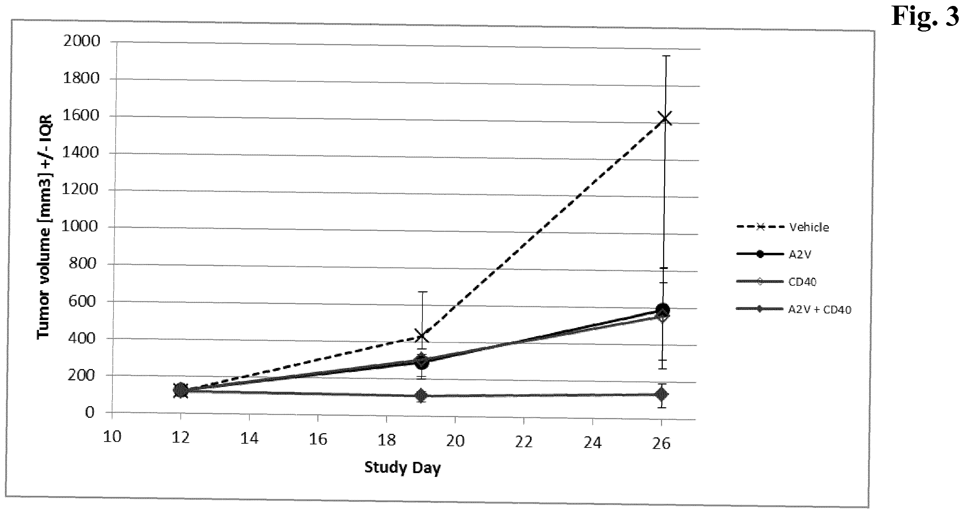

- **Figure 3**: In vivo anti-tumor efficacy of Anti-ANG2 antibodies (bispecific) in combination with an agonistic CD40 antibody in the syngeneic CT26WT model in female Balb/c mice.

### Detailed Description of the Invention

The present invention relates to an anti-ANG2 antibody for use in a method of treatment of cancer in a patient, the method comprising administering the anti-ANG2 antibody and a CD40 agonist to the subject, and the inventors demonstrate herein that this combination of agents results in delayed progression (tumor growth) of cancer and prolonged survival of patients treated).

Human angiopoietin-2 (ANG-2) (alternatively abbreviated with ANGPT2 or ANG2) (SEQ ID No: 15) is described in Maisonpierre, P.C., et al., Science 277 (1997) 55-60 and Cheung, A.H., et al, Genomics 48 (1998) 389-91. The angiopoietins-1 and -2 (ANG-1(SEQ ID No: 108) and ANG-2 (SEQ ID No: 107) were discovered as ligands for the Ties, a family of tyrosine kinases that is selectively expressed within the vascular endothelium. Yancopoulos, G.D., et al., Nature 407 (2000) 242-48. There are now four definitive members of the angiopoietin family. Angiopoietin-3 and -4 (Ang-3 and Ang-4) may represent widely diverged counterparts of the same gene locus in mouse and man. Kim, I., et al., FEBS Let, 443 (1999) 353-56; Kim, I., et al., J Biol Chem 274 (1999) 26523-28. ANG-1 and ANG-2 were originally identified in tissue culture experiments as agonist and antagonist, respectively (see for ANG-1: Davies, S., et al., Cell, 87 (1996) 1161-1169; and for ANG-2: Maisonpierre, P.C., et al., Science 277 (1997) 55-60). All of the known angiopoietins bind primarily to Tie2, and both Ang-1 and -2 bind to Tie2 with an affinity of 3 nM (Kd). Maisonpierre, P.C., et al., Science 277 (1997) 55-60. Ang-1 was shown to support EC survival and to promote endothelium integrity, Davis, S., et al., Cell, 87 (1996) 1161-1169; Kwak, H.J., et al., FEBS Lett 448 (1999) 249-53; Suri, C., et al., Science 282 (1998) 468-71; Thurston, G., et al., Science 286 (1999) 2511-14; Thurston, G., et al., Nat. Med. 6 (2000) 460-63, whereas ANG-2 had the opposite effect and promoted blood vessel destabilization and regression in the absence of the survival factors VEGF or basic fibroblast growth factor. Maisonpierre, P.C., et al., Science 277 (1997) 55-60. However, many studies of ANG-2 function have suggested a more complex situation. ANG-2 might be a complex regulator of vascular remodeling that plays a role in both vessel sprouting and vessel regression. Supporting such roles for ANG-2, expression analyses reveal that ANG-2 is rapidly induced, together with VEGF, in adult settings of angiogenic sprouting, whereas ANG-2 is induced in the absence of VEGF in settings of vascular regression. Holash, J., et al., Science 284 (1999) 1994-98; Holash, J., et al., Oncogene 18 (1999) 5356-62. Consistent with a context-dependent role, ANG-2 specifically binds to the same endothelial-specific receptor, Tie-2, which is activated by Ang-1, but has context-dependent effects on its activation. Maisonpierre, P.C., et al., Science 277 (1997) 55-60.

The term "human ANG2" refers to the human protein angiopoietin 2 (SEQ ID NO: 15). As used herein, "binding to human ANG2" or "specifically binding to human ANG2" or "which binds to human ANG2" or "anti- ANG2 antibody" refers to an antibody specifically binding to the human ANG2 antigen with a binding affinity of KD-value of 1.0 x 10⁻⁸ mol/l or lower, in one embodiment of a KD-value of 1.0 x 10⁻⁹ mol/l or lower. The binding affinity is determined with a standard binding assay, such as surface plasmon resonance technique (BIAcore®, GE-Healthcare Uppsala, Sweden). Thus an "antibody binding to human ANG2" as used herein refers to an antibody specifically binding to the human ANG2 antigen with a binding affinity of KD 1.0 x 10⁻⁸ mol/l or lower (in one embodiment 1.0 x 10⁻⁸ mol/l - 1.0 x 10⁻¹³ mol/l), in on embodiment of a KD 1.0 x 10⁻⁹ mol/l or lower (in one embodiment 1.0 x 10⁻⁹ mol/l - 1.0 x 10⁻¹³ mol/l).

Typically antibodies that bind to human ANG2 which are useful for the treatment described herein are e.g. disclosed and described in detail in WO2010/069532 (e.g. preferably anti-ANG2 antibodies <ANG-2>Ang2i_LC06, <ANG-2>Ang2i_LC07, or <ANG-2> Ang2i_LC10); in WO2011/014469 (e.g. preferably anti-ANG2 antibody H1 H685P); in US2011/150895 (e.g. anti-ANG2 antibodies SAIT-Ang-2-5, SAIT-Ang-2-6 or humanized versions thereof); in WO2009/097325 (e.g. antibody MEDI 1/5 characterized by the VL of MEDI 1 (SEQ ID No:3 in WO 2009/097325) and the VH of (MEDI 5) (SEQ ID No:7 in WO2009/097325)), in WO 2009/105269; in WO 2006/068953 or in WO 03/030833.

In one preferred embodiment the antibody that bind to human ANG2 which are useful for the treatment described herein is characterized in comprising the
(a) a heavy chain variable domain amino acid sequence of SEQ ID NO:1 and a light chain variable domain amino acid sequence of SEQ ID NO:2; or
(b) a heavy chain variable domain amino acid sequence of SEQ ID NO:3 and a light chain variable domain amino acid sequence of SEQ ID NO:4.

In one preferred embodiment the anti-ANG2 antibody is a bispecific antibody that binds to human ANG-2 and that binds to human VEGF.

Typically antibodies bispecific antibody that binds to human ANG-2 and that binds to human VEGF which are useful for the treatment described herein are e.g. disclosed and described in detail in WO2010/040508, WO 2011/117329 or WO2012/131078. Also the VH and VL of the ANG2 antibodies described in WO2010/069532; WO2011/014469; US2011/150895; WO2009/097325; WO2009/105269; WO 2006/068953 or WO 03/030833can be used within the bispecific antibody using the anti-VEGF binding arms and structures described in WO2010/040508, WO 2011/117329 or WO2012/131078.

In one preferred embodiment,
the bispecific antibody that binds to human ANG-2 and that binds to human VEGF comprises
a heavy chain variable domain amino acid sequence of SEQ ID NO:1 and a light chain variable domain amino acid sequence of SEQ ID NO:2; and
a heavy chain variable domain amino acid sequence of SEQ ID NO:9 and a light chain variable domain amino acid sequence of SEQ ID NO:10.

In one preferred embodiment
the bispecific antibody that binds to human ANG-2 and that binds to human VEGF comprises the amino acid sequences of SEQ ID NO: 11, of SEQ ID NO: 12, of SEQ ID NO: 13, and of SEQ ID NO: 14.

The CD40 antigen is a 50 kDa cell surface glycoprotein which belongs to the Tumor Necrosis Factor Receptor (TNF-R) family. (Stamenkovic et al., EMBO J. 8:1403-10 (1989).) CD40 is expressed in many normal and tumor cell types, including B lymphocytes, dendritic cells, monocytes, macrophages, thymic epithelium, endothelial cells, fibroblasts, and smooth muscle cells. (Paulie S. et al., Cancer Immunol. Immunother. 20:23-8 (1985); Banchereau J. et al., Adv. Exp. Med. & Biol. 378:79-83 (1995); Alderson M. R. et al., J. of Exp. Med. 178:669-74 (1993); Ruggiero G. et al., J. of Immunol. 156:3737-46 (1996); Hollenbaugh D. et al., J. of Exp. Med. 182:33-40 (1995); Yellin M. J. et al., J. of Leukocyte Biol. 58:209-16 (1995); and Lazaar A. L. et al., J. of Immunol. 161:3120-7 (1998).) CD40 is expressed in all B-lymphomas and in 70% of all solid tumors. Although constitutively expressed, CD40 is up-regulated in antigen presenting cells by maturation signals, such as LPS, IL-1beta, IFN-gamma and GM-CSF.

CD40 activation plays a critical role in regulating humoral and cellular immune responses. Antigen presentation without CD40 activation can lead to tolerance, while CD40 signaling can reverse such tolerance, enhance antigen presentation by all antigen presenting cells (APCs), lead to secretion of helper cytokines and chemokines, increase co-stimulatory molecule expression and signaling, and stimulate cytolytic activity of immune cells. CD40 plays a critical role in B cell proliferation, maturation and class switching. (Foy T. M. et al., Ann. Rev. of Immunol. 14:591-617 (1996).) Disruption of the CD40 signaling pathway leads to abnormal serum immunoglobulin isotype distribution, lack of CD4+ T cell priming, and defects in secondary humoral responses. For example, the X-linked hyper-IgM syndrome is a disease associated with a mutation in the human CD40L gene, and it is characterized by the inability of affected individuals to produce antibodies other than those of the IgM isotype, indicating that the productive interaction between CD40 and CD40L is required for an effective immune response.

CD40 engagement by CD40L leads to the association of the CD40 cytoplasmic domain with TRAFs (TNF-R associated factors). (Lee H. H. et al., Proc. Natl. Acad. Sci. USA 96:1421-6 (1999); Pullen S. S. et al., Biochemistry 37:11836-45 (1998); Grammar A. C. et al., J. of Immunol. 161:1183-93 (1998); Ishida T. K. et al., Proc. Acad Acad. Sci. USA 93:9437-42 (1996); Pullen S. S. et al., J. of Biol. Chem. 274:14246-54 (1999)). The interaction with TRAFs can culminate in the activation of both NFkappa B and Jun/AP1 pathways. (Tsukamoto N. et al., Proc. Natl. Acad. Sci. USA 96:1234-9 (1999); Sutherland C. L. et al., J. of Immunol. 162:4720-30 (1999).) Depending on cell type, this signaling leads to enhanced secretion of cytokines such as IL-6 (Jeppson J. D. et al., J. of Immunol. 161:1738-42 (1998); Uejima Y. et al., Int. Arch. of Allergy & Immunol. 110:225-32, (1996), IL-8 (Gruss H. J. et al., Blood 84:2305-14 (1994); von Leoprechting A. et al., Cancer Res. 59:1287-94 (1999); Denfeld R. W. et al., Europ. J. of Immunol. 26:2329-34 (1996)), IL-12 (Cella M. et al., J. of Exp. Med. 184:747-52 (1996); Ferlin W. G. et al., Europ. J. of Immunol. 28:525-31 (1998); Armant M. et al., Europ. J. of Immunol. 26:1430-4 (1996); Koch F. et al., J. of Exp. Med. 184:741-6 (1996); Seguin R. and L. H. Kasper, J. of Infect. Diseases 179:467-74 (1999); Chaussabel D. et al., Infection & Immunity 67:1929-34 (1999)), IL-15 (Kuniyoshi J. S. et al., Cellular Immunol. 193:48-58 (1999)) and chemokines (MIP1alpha, MIP1beta, RANTES, and others) (McDyer J. F. et al., J. of Immunol. 162:3711-7 (1999); Schaniel C. et al., J. of Exp. Med. 188:451-63 (1998); Altenburg A. et al., J. of Immunol. 162:4140-7 (1999); Deckers J. G. et al., J. of the Am. Society of Nephrology 9:1187-93 (1998)), increased expression of MHC class I and II (Santos-Argumedo L. et al., Cellular Immunol. 156:272-85 (1994)), and increased expression of adhesion molecules (e.g., ICAM) (Lee H. H. et al., Proc. Natl. Acad. Sci. USA. 96:1421-6 (1999); Grousson J. et al., Archives of Dermatol. Res. 290:325-30 (1998); Katada Y. et al., Europ. J. of Immunol. 26:192-200 (1996); Mayumi M. et al., J. of Allergy & Clin. Immunol. 96:1136-44 (1995); Flores-Romo L. et al., Immunol. 79:445-51 (1993)) and costimulatory molecules (e.g., B7) (Roy M. et al., Europ. J. of Immunol. 25:596-603 (1995); Jones K. W. and C. J. Hackett, Cellular Immunol. 174:42-53 (1996); Caux C. et al., Journal of Exp. Med. 180:1263-72 (1994); Kiener P. A. et al., J. of Immunol. 155:4917-25 (1995)). Cytokines induced by CD40 engagement enhance T cell survival and activation.

In addition to enhancement of cellular and immune function, the effects of CD40 activation include: cell recruitment and differentiation by chemokines and cytokines; activation of monocytes; increased cytolytic activity of cytolytic T lymphocyte (CTL) and natural killer (NK) cells; induction of apoptosis in CD40 positive tumors; enhancement of immunogenicity of CD40 positive tumors; and tumor-specific antibody production. The role of CD40 activation in cell-mediated immune responses is also well established, and it is reviewed in: Grewal et al., Ann. Rev. of Immunol. 16:111-35 (1998); Mackey et al., J. of Leukocyte Biol. 63:418-28 (1998); and Noelle R. J., Agents & Actions -- Suppl. 49:17-22 (1998).

Studies using a cross-priming model system showed that CD40 activation of APCs can replace helper T cell requirement for the generation of cytolytic T lymphocyte (CTL). (Bennett et al., Nature 393:478-480 (1998).) Evidence from CD40L deficient mice indicates a clear requirement for CD40 signaling in helper T cell priming. (Grewal I. S. et al., Science 273:1864-7 (1996); Grewal I. S. et al., Nature 378:617-20 (1995).) CD40 activation converts otherwise tolerogenic, antigen bearing B cells into competent APCs. (Buhlmann J. E. et al., Immunity 2:645-53 (1995).) CD40 activation induces maturation and differentiation of cord blood progenitors into dendritic cells. (Flores-Romo L. et al., J. of Exp. Med. 185:341-9 (1997); Mackey M. F. et al., J. of Immunol. 161:2094-8 (1998).) CD40 activation also induces differentiation of monocytes into functional dendritic cells. (Brossart P. et al., Blood 92:4238-47 (1998).) Further, CD40 activation enhances cytolytic activity of NK cells through APC-CD40 induced cytokines (Carbone E. et al., J. of Exp. Med. 185:2053-60 (1997); Martin-Fontecha A. et al., J. of Immunol. 162:5910-6 (1999).) These observations indicate that CD40 plays an essential role in the initiation and enhancement of immune responses by inducing maturation of APCs, secretion of helper cytokines, upregulation of costimulatory molecules, and enhancement of effector functions.

The critical role of CD40 signaling in the initiation and maturation of humoral and cytotoxic immune responses makes this system an ideal target for immune enhancement. Such enhancement can be particularly important for mounting effective immune responses to tumor antigens, which are generally presented to the immune system through cross-priming of activated APCs. (Huang A. Y. et al., Ciba Foundation Symp. 187:229-44 (1994); Toes R. E. M. et al., Seminars in Immunol. 10:443-8 (1998); Albert M. L. et al., Nature 392:86-9 (1998); Bennett S. R. et al., J. of Exp. Med. 186:65-70 (1997).)

Several groups have demonstrated the effectiveness of CD40 activation for antitumor responses in vitro and in vivo. (Toes R. E. M. et al., Seminars in Immunol. 10:443-8 (1998).) Two groups, using lung metastatic model of renal cell carcinoma and subcutaneous tumors by virally transformed cells, have independently demonstrated that CD40 activation can reverse tolerance to tumor-specific antigens, resulting in efficient antitumor priming of T cells. (Sotomayor E. M. et al., Nature Medicine 5:780-787 (1999); Diehl L. et al., Nature Medicine 5:774-9 (1999).) Antitumor activity in the absence of immune cells was also reported by CD40L and anti-CD40 antibody treatment in a human breast cancer line model in SCID mice. (Hirano A. et al., Blood 93:2999-3007 (1999).) CD40 activation by anti-CD40 antibody was recently shown to eradicate CD40+ and CD40- lymphoma in mouse models. (French R. R. et al., Nature Medicine 5:548-53 (1999).) Furthermore, previous studies by Glennie and co-workers conclude that signaling activity by anti-CD40 antibodies is more effective for inducing in vivo tumor clearance than other anti-surface marker antibodies capable of recruiting effectors. (Tutt A. L. et al., J. of Immunol. 161:3176-85 (1998).) Consistent with these observations, when anti-CD40 antibodies were tested for activity against CD40+ tumor cells in vivo, most but not all of the tumoricidal activity was associated with CD40 signaling rather than ADCC. (Funakoshi S. et al., J. of Immunotherapy with Emphasis on Tumor Immunol. 19:93-101 (1996).) In another study, bone marrow dendritic cells were treated ex vivo with a variety of agents, and tested for in vivo antitumor activity. These studies demonstrated that CD40L stimulated DCs were the most mature and most effective cells that mounting an antitumor response.

The essential role of CD40 in antitumor immunity has also been demonstrated by comparing responses of wild-type and CD40-/- mice to tumor vaccines. These studies show that CD40-/- mice are incapable of achieving the tumor immunity observed in normal mice. (Mackey M. F. et al., Cancer Research 57:2569-74 (1997).) In another study, splenocytes from tumor bearing mice were stimulated with tumor cells and treated with activating anti-CD40 antibodies ex vivo, and were shown to have enhanced tumor specific CTL activity. (Donepudi M. et al., Cancer Immunol. Immunother. 48:153-164 (1999).) These studies demonstrate that CD40 occupies a critical position in antitumor immunity, in both CD40 positive and negative tumors. Since CD40 is expressed in lymphomas, leukemias, multiple myeloma, a majority of carcinomas of nasopharynx, bladder, ovary, and liver, and some breast and colorectal cancers, activation of CD40 can have a broad range of clinical applications.

The "CD40 agonist" as used herein includes any moiety that agonizes the CD40/CD40L interaction. CD40 as used in this context refers preferably to human CD40, thus the CD40 agonist is preferably an agonist of human CD40. Typically these moieties will be agonistic CD40 antibodies or agonistic CD40L polypeptides. In the present invention the CD40 agonist is an agonistic CD40 agonist antibody. These antibodies include by way of example human antibodies, chimeric antibodies, humanized antibodies, bispecific antibodies, scFvs, and antibody fragments that specifically agonize the CD40/CD40L binding interaction.

In one preferred embodiment the agonistic CD40 antibody will comprise a chimeric, fully human or humanized CD40 antibody. In another preferred embodiment the agonistic CD40 antibody will comprise a chimeric, fully human or humanized CD40 antibody.

An "agonist" combines with a receptor on a cell and initiates a reaction or activity that is similar to or the same as that initiated by a natural ligand of the receptor. An "CD40 agonist" induces any or all of, but not limited to, the following responses: B cell proliferation and/or differentiation; upregulation of intercellular adhesion via such molecules as ICAM- 1, E-selectin, VC AM, and the like; secretion of pro-inflammatory cytokines such as IL-1, IL-6, IL-8, IL-12, TNF, and the like; signal transduction through the CD40 receptor by such pathways as TRAF {e.g., TRAF2 and/or TRAF3), MAP kinases such as NIK (NF-kB inducing kinase), I-kappa B kinases (IKK/.beta.), transcription factor NF-kB, Ras and the MEK/ERK pathway, the PI3K AKT pathway, the P38 MAPK pathway, and the like; transduction of an anti-apoptotic signal by such molecules as XIAP, mcl-1, bcl-x, and the like; B and/or T cell memory generation; B cell antibody production; B cell isotype switching, up-regulation of cell-surface expression of MHC Class II and CD80/86, and the like.

By agonist activity is intended an agonist activity of at least 30%, 10 35%, 40%, 45%, 50%, 60%, 70%, 75%, 80%, 85%, 90%, 95%, or 100% greater than the agonist activity induced by a negative control as measured in an assay of a B cell response.

In one preferred embodiment an CD40 agonist has an agonist activity that is at least 2- fold greater or at least 3-fold greater than the agonist activity induced by a negative control as measured in an assay of a B cell response.

Thus, for example, where the B cell response of interest is B cell proliferation, agonist activity would be induction of a level of B cell proliferation that is at least 2-fold greater or at least 3-fold greater than the level of B cell proliferation induced by a negative control.

In one embodiment, an antibody that does not bind to CD40 serves as the negative control. A substance "free of significant agonist activity" would exhibit an agonist activity of not more than about 25% greater than the agonist activity induced by a negative control, preferably not more than about 20% greater, 15% greater, 10% greater, 5% greater, 1 % greater, 0.5% greater, or even not more than about 0.1% greater than the agonist activity induced by a negative control as measured in an assay of a B cell response.

An "agonistic CD40 antibody", or "activating CD40 antibody" or "agonistic or activating anti-CD40 antibody" as used herein means an antibody that binds to human CD40 and that increases one or more CD40 activities by at least about 20% when added to a cell, tissue or organism expressing CD40. In some embodiments, the antibody activates CD40 activity by at least 40%, 50%, 60%, 70%, 80%, 85%.

In some embodiments, the activating antibody is added in the presence of CD40L.

In another preferred embodiment, the agonist activity of the agonistic CD40 antibody is measured as follows:

### Increase of Immunogenicity of Cell Line Jy by Anti-CD40 Antibodies

CD40 positive JIYOYE cells (ATCC CCL 87) ("Jy cells") were cultured and maintained in RPMI medium. JIYOYE cells were incubated for 24 hours with an anti-CD40 antibody of the invention (21.4.1), or with an isotype matched antibody (anti-KLH), in complete RPMI medium. Cells were then washed and treated with 25 mg mitomycin C (Sigma)/7 ml media for 60 min. These cells were then incubated with isolated human T cells at a 1:100 ratio for 6 days at 37 deg. C. (5% CO2). T cells were then collected, washed, and the level of CTL activity determined against fresh chromium 51 (New England Nuclear, Boston, Mass.) labeled JIYOYE cells. Specific CTL activity was calculated as % specific cytolysis=(cytolysis Jy (cpm)-spontaneous cytolysis (cpm))/(total cytolysis (cpm)-spontaneous cytolysis (cpm)).

In one preferred embodiment the agonistic CD40 antibody as used herein inreases immunogenicity in CD40 positive JIYOYE cells (ATCC CCL 87) by at least 50% as measured in (above described) in vitro JIYOYE cells (ATCC CCL 87) assay.

Agonistic CD40 antibodies also named anti-CD40 activating antibodies herein can contribute to tumor eradication via several important mechanisms. Foremost among these is activation of host dendritic cells for enhanced tumor antigen processing and presentation, as well as enhanced antigen presentation or immunogenicity of CD40 positive tumor cells themselves, leading to activation of tumor specific CD4+ and CD8+ lymphocytes. Additional antitumor activity can be mediated by other immune-enhancing effects of CD40 signaling (production of chemokines and cytokines, recruitment and activation monocytes, and enhanced CTL and NK cytolytic activity), as well as direct killing of CD40+ tumors by induction of apoptosis or by stimulating a humoral response leading to ADCC. Apoptotic and dying tumor cells can also become an important source of tumor-specific antigens that are processed and presented by CD40 activated APCs.

The present invention describes an isolated antibody or antigen-binding portion thereof that binds human CD40 and acts as a CD40 agonist.

Agonistic CD40 antibodies are described e.g. Beatty et al., Science 331 (2011) 1612-1616, R. H. Vonderheide et al., J Clin Oncol 25, 876 (2007); Khalil, M, et al., Update Cancer Ther. 2007 June 1; 2(2): 61-65, an agonist CD40 rat anti-mouse IgG2a mAb FGK45 as model antibody is described in S. P. Schoenberger, et al, Nature 393, 480 (1998)); the mouse cross-reactive agonistic CD40 antibody Clone 1C10 is described in Santos-Argumedo L. et al., Cell Immunol. 156 (1994) 272-285 and Heath AW et al. Eur J Immunol 24 (1994) 1828-34. Examples in clinical trials are e.g. CP-870,893 and dacetuzumab (an agonist CD40 antibody, CAS number 880486-59-9, SGN-40; humanized S2C6 antibody) (Khalil, M, et al, Update Cancer Ther. 2007 June 1; 2(2): 61-65.

In one preferred embodiment the agonistic CD40 antibody is CP-870,893 which a fully human IgG2 agonistic CD40 antibody developed by Pfizer. It binds human CD40 with a KD of 3.48×10⁻¹⁰ M, but does not block binding of CD40L (see e.g., U.S.7,338,660 or EP1476185 wherein CP-870,893 is described as antibody 21.4.1). CP-870,893 (antibody 21.4.1 of US 7,338,660) is characterized by comprising (a) a heavy chain variable domain amino acid sequence of QVQLVQSGAEVKKPGASVKVSCKAS GYTFTGYYMHWVRQAPGQGLEWMGWINPDSGGTNYAQKFQGRVTMTR DTSISTAYMELNRLRSDDTAVYYCARDQPLGYCTNGVCSYFDYWGQGTL VTVSS (SEQ ID NO: 5) (which corresponds to SEQ ID NO: 42 of US 7,338,660) (b) a light chain variable domain amino acid sequence of DIQMTQSPSSVSASVGDRVTITCRASQGIYSWLAWYQQKPGKAPNLLIYTA STLQSGVPSRFSGSGSGTDFTLTISSLQPEDFATYYCQQANIFPLTFGGGTKV EIK (SEQ ID NO: 6) (which corresponds to SEQ ID NO: 44 of US 7,338,660); and /or having the heavy chain variable domain and light chain variable domain amino acid sequences of the antibody produced by hybridoma 21.4.1 having American Type Culture Collection (ATCC) accession number PTA-3605. Dacetuzumab and other humanized S2C6 antibodies are described in US 6,946,129 and US 8,303,955.

Therefore in one preferred embodiment the agonistic CD40 antibody used in the combination therapy with an ANG-2 or an ANG-2/VEGF antibody is characterized by comprising (a) a heavy chain variable domain amino acid sequence of QVQLVQSGAEVKKPGASVKVSCKAS GYTFTGYYMHWVRQAPGQGLEWMGWINPDSGGTNYAQKFQGRVTMTR DTSISTAYMELNRLRSDDTAVYYCARDQPLGYCTNGVCSYFDYWGQGTL VTVSS (SEQ ID NO: 5) (which corresponds to SEQ ID NO: 42 of US 7,338,660) (b) a light chain variable domain amino acid sequence of DIQMTQSPSSVSASVGDRVTITCRASQGIYSWLAWYQQKPGKAPNLLIYTA STLQSGVPSRFSGSGSGTDFTLTISSLQPEDFATYYCQQANIFPLTFGGGTKV EIK (SEQ ID NO: 6) (which corresponds to SEQ ID NO: 44 of US 7,338,660); and /or having the heavy chain variable domain and light chain variable domain amino acid sequences of the antibody produced by hybridoma 21.4.1 having American Type Culture Collection (ATCC) accession number PTA-3605. Dacetuzumab and other humanized S2C6 antibodies are described in US 6,946,129 and US 8,303,955.

In one preferred embodiment the agonistic CD40 antibody used in the combination therapy with an ANG-2 or an ANG-2/VEGF antibody is a humanized S2C6 antibody. A humanized S2C6 antibody is e.g. based on the CDR1, 2 and 3 of the heavy and light chain variable domain of murine mAB S2C6 (deposited with the ATCC as PTA-110). The CDR1, 2 and 3 of the heavy and light chain variable domain of murine mAB S2C6 is described and disclosed US 6,946,129. In one embodiment the agonist CD40 antibody is dacetuzumab. In one embodiment the agonist CD40 antibody is characterized by comprising (a) a heavy chain variable domain amino acid sequence of EVQLVESGGGLVQPGGSLRLSCAASGYSFTGYYIHWVRQAPGKGLEWVA RVIPNAGGTSYNQKFKGRFTLSVDNSKNTAYLQMNSLRAEDTAVYYCARE GIYWWGQGTLVTVS (SEQ ID NO: 7) (b) a light chain variable domain amino acid sequence of DIQMTQSPSSLSASVGDRVTITCRSSQSLVHSNGNTFLHW YQQKPGKAPKLLIYTVSNRFSGVPSRFSGSGSGTDFTLTISSLQPEDFAT YFCSQTTHVPWTFGQGTKVEIKR (SEQ ID NO: 8).

In one embodiment the agonist CD40 antibody is a human antibody. As used herein, the term "human antibody" means an antibody in which the variable and constant domain sequences are derived from human sequences. Human agonistic CD40 antibodies are described in detail in WO 03/040170 the entire disclosure of which is hereby incorporated by reference. Human antibodies provide a substantial advantage in the treatment methods of the present invention, as they are expected to minimize the immunogenic and allergic responses that are associated with use of non-human antibodies in human patients.

Other Exemplary human anti-CD40 antibodies useful for the present invention include antibodies having the amino acid sequences of antibodies designated 3.1.1, 3.1.1 H-A78T, 3.1.1 H-A78T-V88A-V97A, 7.1.2, 10.8.3, 15.1.1, 21.4.1, 21.2.1, 22.1.1, 22.1.1 H-C109A, 23.5.1, 23.25.1, 23.28.1, 23.28.1H-D16E, 23.29.1, 24.2.1, 3.1.1H-A78T-V88A-V97A/3.1.1L-L4M-L83V and 23.28.1L-C92A, described in WO03/040170 as well as an antibody comprising a CDR or variable region of any of the exemplary antibodies.

In certain embodiments, the cancer or tumor treatment inhibits cancer cell proliferation, inhibits or prevents an increase in tumor weight or volume, and/or causes a decrease in tumor weight or volume. In some embodiments, the cancer treatment prolongs patient survival. In certain embodiments, tumor growth is inhibited at least 50%, 55%, 60%, 65%, 70% or 75%, compared to those not treated. In some embodiments, the cancer or tumor is CD40 positive.

In the present invention the term "CD40L" or "CD154" as it alternatively known in the art includes all mammalian CD40L's, e.g., human, rat, non-human primate, murine as well as fragments, variants, oligomers, and conjugates thereof that bind to at least the corresponding mammalian CD40 polypeptide, e.g., human CD40. In the present invention the administered CD40L may comprise a CD40L polypeptide or a DNA encoding said CD40L polypeptide. Such CD40L polypeptides and DNAs include in particular native CD40L sequences and fragments, variants, and oligomers thereof as disclosed in Immunex U.S. Pat. No. 6,410,711; U.S. Pat. No. 6,391,637; U.S. Pat. No. 5,981,724; U.S. Pat. No. 5,961,974 and US published application No. 20040006006.

The CD40L polypeptide can be used as CD40 agonist and includes in particular native CD40L sequences and fragments, variants , and oligomers thereof as disclosed in Immunex U.S. Pat. No. 6,410,711; U.S. Pat. No. 6,391,637; U.S. Pat. No. 5,981,724; U.S. Pat. No. 5,961,974 and US published application No. 20040006006.

The term "epitope" denotes a protein determinant of the antigen capable of specifically binding to an antibody. Epitopes usually consist of chemically active surface groupings of molecules such as amino acids or sugar side chains and usually epitopes have specific three dimensional structural characteristics, as well as specific charge characteristics. Conformational and nonconformational epitopes are distinguished in that the binding to the former but not the latter is lost in the presence of denaturing solvents.

The "variable domain" (light chain variable domain VL, heavy chain variable domain VH) as used herein denotes each of the pair of light and heavy chain domains which are involved directly in binding the antibody to the antigen. The variable light and heavy chain domains have the same general structure and each domain comprises four framework (FR) regions whose sequences are widely conserved, connected by three "hypervariable regions" (or complementary determining regions, CDRs). The framework regions adopt a beta-sheet conformation and the CDRs may form loops connecting the beta-sheet structure. The CDRs in each chain are held in their three-dimensional structure by the framework regions and form together with the CDRs from the other chain the antigen binding site. The antibody's heavy and light chain CDR3 regions play a particularly important role in the binding specificity/affinity of the antibodies according to the invention and therefore provide a further object of the invention.

The term "antigen-binding portion of an antibody" when used herein refer to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD (1991) and/or those residues from a "hypervariable loop".

The terms "nucleic acid" or "nucleic acid molecule", as used herein, are intended to include DNA molecules and RNA molecules. A nucleic acid molecule may be single-stranded or double-stranded, but preferably is double-stranded DNA.

The term "amino acid" as used within this application denotes the group of naturally occurring carboxy alpha-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

The "Fc part" of an antibody is not involved directly in binding of an antibody to an antigen, but exhibit various effector functions. A "Fc part of an antibody" is a term well known to the skilled artisan and defined on the basis of papain cleavage of antibodies. Depending on the amino acid sequence of the constant region of their heavy chains, antibodies or immunoglobulins are divided in the classes: IgA, IgD, IgE, IgG and IgM, and several of these may be further divided into subclasses (isotypes), e.g. IgG1, IgG2, IgG3, and IgG4, IgA1, and IgA2. According to the heavy chain constant regions the different classes of immunoglobulins are called α, δ, ε, γ, and µ, respectively. The Fc part of an antibody is directly involved in ADCC (antibody-dependent cell-mediated cytotoxicity) and CDC (complement-dependent cytotoxicity) based on complement activation, C1q binding and Fc receptor binding. Complement activation (CDC) is initiated by binding of complement factor C1q to the Fc part of most IgG antibody subclasses. While the influence of an antibody on the complement system is dependent on certain conditions, binding to C1q is caused by defined binding sites in the Fc part. Such binding sites are known in the state of the art and described e.g. by Boackle, R.J., et al., Nature 282 (1979) 742-743; Lukas, T.J., et al., J. Immunol. 127 (1981) 2555-2560; Brunhouse, R., and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol.164 (2000) 4178-4184; Hezareh, M., et al., J. Virology 75 (2001) 12161-12168; Morgan, A., et al., Immunology 86 (1995) 319-324; EP 0 307 434. Such binding sites are e.g. L234, L235, D270, N297, E318, K320, K322, P331 and P329 (numbering according to EU index of Kabat, E.A., see below). Antibodies of subclass IgG1, IgG2 and IgG3 usually show complement activation and C1q and C3 binding, whereas IgG4 do not activate the complement system and do not bind C1q and C3.

In one embodiment the antibodies described herein are of human IgG class (i.e. of IgG1, IgG2, IgG3 or IgG4 subclass).

In a preferred embodiment the antibodies described herein are of human IgG1 subclass or of human IgG4 subclass. In one embodiment the described herein are of human IgG1 subclass. In one embodiment the antibodies described herein are of human IgG4 subclass.

In one embodiment the antibody according to the invention comprises an Fc part derived from human origin and preferably all other parts of the human constant regions. As used herein the term "Fc part derived from human origin" denotes a Fc part which is either a Fc part of a human antibody of the subclass IgG1, IgG2, IgG3 or IgG4, preferably a Fc part from human IgG1 subclass, a mutated Fc part from human IgG1 subclass (in one embodiment with a mutation on L234A + L235A), a Fc part from human IgG4 subclass or a mutated Fc part from human IgG4 subclass (in one embodiment with a mutation on S228P).

In one embodiment the antibody described herein is characterized in that the constant chains are of human origin. Such constant chains are well known in the state of the art and e.g. described by Kabat, E.A., (see e.g. Johnson, G. and Wu, T.T., Nucleic Acids Res. 28 (2000) 214-218).

The antibodies described herein are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression nucleic acids encoding light and heavy chains or fragments thereof are inserted into expression vectors by standard methods. Expression is performed in appropriate prokaryotic or eukaryotic host cells, such as CHO cells, NS0 cells, SP2/0 cells, HEK293 cells, COS cells, yeast, or E. coli cells, and the antibody is recovered from the cells (from the supernatant or after cells lysis).

Recombinant production of antibodies is well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

The antibodies may be present in whole cells, in a cell lysate, or in a partially purified, or substantially pure form. Purification is performed in order to eliminate other cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987).

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; Norderhaug, L., et al., J. Immunol. Methods 204 (1997) 77-87. A preferred transient expression system (HEK 293) is described by Schlaeger, E.-J. and Christensen, K., in Cytotechnology 30 (1999) 71-83, and by Schlaeger, E.-J., in J. Immunol. Methods 194 (1996) 191-199.

The heavy and light chain variable domains according to the invention are combined with sequences of promoter, translation initiation, constant region, 3' untranslated region, polyadenylation, and transcription termination to form expression vector constructs. The heavy and light chain expression constructs can be combined into a single vector, co-transfected, serially transfected, or separately transfected into host cells which are then fused to form a single host cell expressing both chains.

The control sequences that are suitable for prokaryotes, for example, include a promoter, optionally an operator sequence, and a ribosome binding site. Eukaryotic cells are known to utilize promoters, enhancers and polyadenylation signals.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The monoclonal antibodies are suitably separated from the culture medium by conventional immunoglobulin purification procedures such as, for example, protein A-Sepharose, hydroxylapatite chromatography, gel electrophoresis, dialysis, or affinity chromatography. DNA and RNA encoding the monoclonal antibodies are readily isolated and sequenced using conventional procedures. The hybridoma cells can serve as a source of such DNA and RNA. Once isolated, the DNA may be inserted into expression vectors, which are then transfected into host cells such as HEK 293 cells, CHO cells, or myeloma cells that do not otherwise produce immunoglobulin protein, to obtain the synthesis of recombinant monoclonal antibodies in the host cells.

As used herein, the expressions "cell", "cell line", and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included.

In another aspect, the present invention provides a composition, e.g. a pharmaceutical composition, containing one or a combination of monoclonal antibodies, or the antigen-binding portion thereof, of the present invention, formulated together with a pharmaceutically acceptable carrier.

As used herein, "pharmaceutically acceptable carrier" includes any and all solvents, dispersion media, coatings, antibacterial and antifungal agents, isotonic and absorption/resorption delaying agents, and the like that are physiologically compatible. Preferably, the carrier is suitable for injection or infusion.

A composition of the present invention can be administered by a variety of methods known in the art. As will be appreciated by the skilled artisan, the route and/or mode of administration will vary depending upon the desired results.

Pharmaceutically acceptable carriers include sterile aqueous solutions or dispersions and sterile powders for the preparation of sterile injectable solutions or dispersion. The use of such media and agents for pharmaceutically active substances is known in the art. In addition to water, the carrier can be, for example, an isotonic buffered saline solution.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient (effective amount). The selected dosage level will depend upon a variety of pharmacokinetic factors including the activity of the particular compositions of the present invention employed, or the ester, salt or amide thereof, the route of administration, the time of administration, the rate of excretion of the particular compound being employed, other drugs, compounds and/or materials used in combination with the particular compositions employed, the age, sex, weight, condition, general health and prior medical history of the patient being treated, and like factors well known in the medical arts.

The term "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The terms "administered in combination with" or "co-administration", "coadministering", "combination therapy", " administered with" or "combination treatment" refer to the administration of the anti-ANG2 antibody as described herein, and the agonistic CD40 antibody as described herein e.g. as separate formulations/applications (or as one single formulation/application). The co-administration can be simultaneous or sequential in either order, wherein preferably there is a time period while both (or all) active agents simultaneously exert their biological activities. Said antibody and said further agent are co-administered either simultaneously or sequentially (e.g. intravenous (i.v.) through a continuous infusion. When both therapeutic agents are co-administered sequentially the dose is administered either on the same day in two separate administrations, or one of the agents is administered on day 1 and the second is co-administered on day 2 to day 7, preferably on day 2 to 4. Thus in one embodiment the term "sequentially" means within 7 days after the dose of the first component, preferably within 4 days after the dose of the first component; and the term "simultaneously" means at the same time. The terms "co-administration" with respect to the maintenance doses of anti-ANG2 antibody and/or agonistic CD40 antibody mean that the maintenance doses can be either co-administered simultaneously, if the treatment cycle is appropriate for both drugs, e.g. every week. Or the further agent is e.g. administered e.g. every first to third day and said antibody is administered every week. Or the maintenance doses are co-administered sequentially, either within one or within several days.

It is self-evident that the antibodies are administered to the patient in a "therapeutically effective amount" (or simply "effective amount") which is the amount of the respective compound or combination that will elicit the biological or medical response of a tissue, system, animal or human that is being sought by the researcher, veterinarian, medical doctor or other clinician.

As used herein, the term "patient" or "subject" preferably refers to a human in need of treatment of cancer, or a precancerous condition or lesion. However, the term "patient" can also refer to non-human animals, e.g. mammals such as mice, dogs, cats, horses, cows, pigs, sheep and non-human primates, among others, that are in need of treatment.

The amount of co-administration and the timing of co-administration will depend on the type (species, gender, age, weight, etc.) and condition of the patient being treated and the severity of the disease or condition being treated. Said anti-ANG2 antibody and further agent are suitably co-administered to the patient at one time or over a series of treatments e.g. on the same day or on the day after.

Depending on the type and severity of the disease, about 0.1 mg /kg to 50 mg/kg (e.g. 0.1-20 mg/kg) of said anti-ANG2 antibody and/or agonistic CD40 antibody; is an initial candidate dosage for co-administration of both drugs to the patient The invention comprises the use of the antibodies according to the invention for the treatment of a patient suffering from cancer, especially from colon cancer, ovarian cancer, glioblastoma, gastric cancer, pancreatic cancer, breast cancer, lung cancer, hepatocellular cancer.

In addition to the anti-ANG2 antibody in combination with the agonistic CD40 antibody also a chemotherapeutic agent can be administered.

In one embodiment such additional chemotherapeutic agents, which may be administered with anti-ANG2 antibody as described herein and the agonistic CD40 antibody as described herein , include, but are not limited to, anti-neoplastic agents including alkylating agents including: nitrogen mustards, such as mechlorethamine, cyclophosphamide, ifosfamide, melphalan and chlorambucil; nitrosoureas, such as carmustine (BCNU), lomustine (CCNU), and semustine (methyl-CCNU); Temodal(TM) (temozolamide), ethylenimines/methylmelamine such as thriethylenemelamine (TEM), triethylene, thiophosphoramide (thiotepa), hexamethylmelamine (HMM, altretamine); alkyl sulfonates such as busulfan; triazines such as dacarbazine (DTIC); antimetabolites including folic acid analogs such as methotrexate and trimetrexate, pyrimidine analogs such as 5-fluorouracil (5FU), fluorodeoxyuridine, gemcitabine, cytosine arabinoside (AraC, cytarabine), 5-azacytidine, 2,2'-difluorodeoxycytidine, purine analogs such as 6-merca.rho.topurine, 6-thioguamne, azathioprine, T-deoxycoformycin (pentostatin), erythrohydroxynonyladenine (EHNA), fludarabine phosphate, and 2-chlorodeoxyadenosine (cladribine, 2-CdA); natural products including antimitotic drugs such as paclitaxel, vinca alkaloids including vinblastine (VLB), vincristine, and vinorelbine, taxotere, estramustine, and estramustine phosphate; pipodophylotoxins such as etoposide and teniposide; antibiotics such as actinomycin D, daunomycin (rubidomycin), doxorubicin, mitoxantrone, idarubicin, bleomycins, plicamycin (mithramycin), mitomycin C, and actinomycin; enzymes such as L-asparaginase; biological response modifiers such as interferon-alpha, IL-2, G-CSF and GM-CSF; miscellaneous agents including platinum coordination complexes such as oxaliplatin, cisplatin and carboplatin, anthracenediones such as mitoxantrone, substituted urea such as hydroxyurea, methylhydrazine derivatives including N- methylhydrazine (MIH) and procarbazine, adrenocortical suppressants such as mitotane (o, p-DDD) and aminoglutethimide; hormones and antagonists including adrenocorticosteroid antagonists such as prednisone and equivalents, dexamethasone and aminoglutethimide; Gemzar(TM) (gemcitabine), progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide. Therapies targeting epigenetic mechanism including, but not limited to, histone deacetylase inhibitors, demethylating agents (e.g., Vidaza) and release of transcriptional repression (ATRA) therapies can also be combined with the antigen binding proteins. In one embodiment the chemotherapeutic agent is selected from the group consisting of taxanes (like e.g. paclitaxel (Taxol), docetaxel (Taxotere), modified paclitaxel (e.g., Abraxane and Opaxio), doxorubicin, sunitinib (Sutent), sorafenib (Nexavar), and other multikinase inhibitors, oxaliplatin, cisplatin and carboplatin, etoposide, gemcitabine, and vinblastine. In one embodiment the chemotherapeutic agent is selected from the group consisting of taxanes (like e.g. taxol (paclitaxel), docetaxel (Taxotere), modified paclitaxel (e.g. Abraxane and Opaxio). In one embodiment, the additional chemotherapeutic agent is selected from 5-fluorouracil (5-FU), leucovorin, irinotecan, or oxaliplatin. In one embodiment the chemotherapeutic agent is 5-fluorouracil, leucovorin and irinotecan (FOLFIRI). In one embodiment the chemotherapeutic agent is 5-fluorouracil, and oxaliplatin (FOLFOX).

Specific examples of combination therapies with additional chemotherapeutic agents include, for instance, therapies taxanes (e.g., docetaxel or paclitaxel) or a modified paclitaxel (e.g., Abraxane or Opaxio), doxorubicin), capecitabine and/or bevacizumab (Avastin) for the treatment of breast cancer; therapies with carboplatin, oxaliplatin, cisplatin, paclitaxel, doxorubicin (or modified doxorubicin (Caelyx or Doxil)), or topotecan (Hycamtin) for ovarian cancer, the therapies with a multi-kinase inhibitor, MKI, (Sutent, Nexavar, or 706) and/or doxorubicin for treatment of kidney cancer; therapies with oxaliplatin, cisplatin and/or radiation for the treatment of squamous cell carcinoma; therapies with taxol and/or carboplatin for the treatment of lung cancer.

Therefore, in one embodiment the additional chemotherapeutic agent is selected from the group of taxanes (docetaxel or paclitaxel or a modified paclitaxel (Abraxane or Opaxio), doxorubicin, capecitabine and/or bevacizumab for the treatment of breast cancer.

In one embodiment of the anti-ANG2 antibody/agonistic CD40 antibody combination therapy, no additional chemotherapeutic agents are administered.

The invention comprises also a method for the treatment of a patient suffering from such disease.

The invention further provides a method for the manufacture of a pharmaceutical composition comprising an effective amount of an antibody according to the invention together with a pharmaceutically acceptable carrier and the use of the antibody according to the invention for such a method.

The invention further provides the use of an antibody according to the invention in an effective amount for the manufacture of a pharmaceutical agent, preferably together with a pharmaceutically acceptable carrier, for the treatment of a patient suffering from cancer.

The invention also provides the use of an antibody according to the invention in an effective amount for the manufacture of a pharmaceutical agent, preferably together with a pharmaceutically acceptable carrier, for the treatment of a patient suffering from cancer.

### The following is disclosed herein:

1. An antibody that binds to human angiopoietin 2 (ANG-2) wherein the antibody is administered in combination with CD40 agonist a) for use in treating or delaying progression of cancer, or b) for use in prolonging the survival of a patient suffering from cancer, or c) for use in stimulating an immune response or function, such as T cell activity (in one embodiment CD8 effector T cell activity) or macrophage activity (in one embodiment CD40-activated macrophage activity), or d) for use in rendering a cancer susceptible for the treatment with an antibody that binds to human angiopoietin 2 (ANG-2).
2. An antibody that binds to human angiopoietin 2 (ANG-2) wherein the antibody is administered in combination with CD40 agonist a) for use in treating or delaying progression of cancer, b) for use in prolonging the survival of a patient suffering from cancer.
3. The anti-ANG2 antibody for use according to embodiment 1 or 2, wherein the anti-ANG2 antibody is a monoclonal antibody.
4. The anti-ANG2 antibody for use according to any one of the preceding embodiments, wherein the anti-ANG2 antibody is human or humanized.
5. The anti-ANG2 antibody for use according to any one of the preceding embodiments, wherein the anti-ANG2 antibody specifically binds to human ANG2 with a K_{D} value of less than 1.0 x 10⁻⁸ mol/l, as determined by surface plasmon resonance (Biacore™).
6. The anti-ANG2 antibody for use according to any one of the preceding embodiments, wherein the anti-ANG2 antibody is an IgG antibody.
7. The anti-ANG2 antibody for use according to any one of the preceding embodiments, wherein the anti-ANG2 antibody inhibits the interaction of human ANG-2 with TIE2 receptor with an IC50 of 15 nM or less.
8. The anti-ANG2 antibody for use according to any one of the preceding embodiments, wherein the CD40 agonist is an agonistic CD40 antibody or an agonistic CD40L polypeptide.
9. The anti-ANG2 antibody for use according to any one of the preceding embodiments, wherein the CD40 agonist is an agonistic CD40 antibody.
10. The antibody that binds to human ANG-2 according to embodiment 9,
   i) wherein the anti-ANG2 antibody comprises
      (a) a heavy chain variable domain amino acid sequence of SEQ ID NO:1 and a light chain variable domain amino acid sequence of SEQ ID NO:2; or
      (b) a heavy chain variable domain amino acid sequence of SEQ ID NO:3 and a light chain variable domain amino acid sequence of SEQ ID NO:4;
      and
   ii) wherein the agonistic CD40 antibody comprises
      (a) a heavy chain variable domain amino acid sequence of SEQ ID NO: 5 and a light chain variable domain amino acid sequence of SEQ ID NO: 6; or
      (b) a heavy chain variable domain amino acid sequence of SEQ ID NO: 7 and a light chain variable domain amino acid sequence of SEQ ID NO: 8;
11. The antibody that binds to human ANG-2 according to embodiment 9,
   i) wherein the anti-ANG2 antibody comprises
      a heavy chain variable domain amino acid sequence of SEQ ID NO:1 and a light chain variable domain amino acid sequence of SEQ ID NO:2; or
      and
   ii) wherein the agonistic CD40 antibody comprises
   a heavy chain variable domain amino acid sequence of SEQ ID NO: 5 and a light chain variable domain amino acid sequence of SEQ ID NO: 6.
12. The anti-ANG2 antibody for use according to embodiment 9, wherein the anti-ANG2 antibody is a bispecific antibody that binds to human ANG-2 and that binds to human VEGF.
13. The antibody that binds to human ANG-2 according embodiment 10,
   i) wherein the bispecific antibody that binds to human ANG-2 and that binds to human VEGF comprises
      a heavy chain variable domain amino acid sequence of SEQ ID NO:1 and a light chain variable domain amino acid sequence of SEQ ID NO:2; and
      a heavy chain variable domain amino acid sequence of SEQ ID NO:9 and a light chain variable domain amino acid sequence of SEQ ID NO:10;
      and
   ii) wherein the agonistic CD40 antibody comprises
   a heavy chain variable domain amino acid sequence of SEQ ID NO: 5 and a light chain variable domain amino acid sequence of SEQ ID NO: 6.
14. The antibody that binds to human ANG-2 according embodiment 10,
   i) wherein the bispecific antibody that binds to human ANG-2 and that binds to human VEGF comprises the amino acid sequences of SEQ ID NO: 11, of SEQ ID NO: 12, of SEQ ID NO: 13, and of SEQ ID NO: 14; and
   ii) wherein the agonistic CD40 antibody comprises
   a heavy chain variable domain amino acid sequence of SEQ ID NO: 5 and a light chain variable domain amino acid sequence of SEQ ID NO: 6.
15. An antibody that binds to human angiopoietin 2 (ANG-2) for use according to any one of the preceding embodiments, wherein the cancer is lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma or lymphocytic leukemia.
16. An antibody that binds to human angiopoietin 2 (ANG-2) for use according to embodiment 15, wherein the cancer comprises a solid tumor.
17. An antibody that binds to human angiopoietin 2 (ANG-2) for use according to embodiment 15 or 16, wherein the cancer is colon cancer, ovarian cancer, glioblastoma, gastric cancer, pancreatic cancer, breast cancer, lung cancer, hepatocellular cancer.
18. The antibody that binds to human angiopoietin 2 (ANG-2) for use according to any one of the preceding embodiments, wherein in the method of treatment, the anti-ANG2 antibody and the CD40 agonist are administered to the subject simultaneously, separately or sequentially.

### Description of the amino acid sequences

| | |
|---|---|
| SEQ ID NO: 1 | variable heavy chain domain VH of <ANG-2> E6Q |
| SEQ ID NO: 2 | variable light chain domain VL of <ANG-2> E6Q |
| SEQ ID NO: 3 | variable heavy chain domain VH of <ANG-2> Ang2i_LC06 |
| SEQ ID NO: 4 | variable light chain domain VL of < ANG-2> Ang2i_LC06 |
| SEQ ID NO: 5 | variable heavy chain domain VH of CP-870,893 (antibody 21.4.1 of U.S.7,338,660) |
| SEQ ID NO: 6 | variable light chain domain VL of CP-870,893 (antibody 21.4.1 of U.S.7,338,660) |
| SEQ ID NO: 7 | humanized S2C6 heavy chain variable domain VH variant |
| SEQ ID NO: 8 | humanized S2C6 light chain variable domain VL variant |
| SEQ ID NO: 9 | variable heavy chain domain VH of <VEGF> bevacizumab |
| SEQ ID NO: 10 | variable light chain domain VL of <VEGF> bevacizumab |
| SEQ ID NO: 11 | Bispecific ANG2/VEGF antibody XMab1 -<VEGF> light chain |
| SEQ ID NO: 12 | Bispecific ANG2/VEGF antibody XMab1 -<ANG2> light chain |
| SEQ ID NO: 13 | Bispecific ANG2/VEGF antibody XMab1 -<VEGF> heavy chain |
| SEQ ID NO: 14 | Bispecific ANG2/VEGF antibody XMab1 -<ANG2> heavy chain |
| SEQ ID NO: 15 | Human angiopoietin-2 (ANG-2) |
| SEQ ID NO: 16 | Human vascular endothelial growth factor (VEGF) |
| SEQ ID NO: 17 | variable heavy chain domain VH of <VEGF> B20-4.1 |
| SEQ ID NO: 18 | variable light chain domain VL of <VEGF> B20-4.1 |

In the following embodiments of the invention are described:
1. An antibody that binds to human angiopoietin 2 (ANG-2) a) for use in a method of treating or delaying progression of cancer, or b) for use in a method of prolonging the survival of a patient suffering from cancer, wherein the method comprises administration of said antibody in combination with an agonistic CD40 antibody.
2. The antibody for use according to claim 1 for use in stimulating an immune response or function, such as T cell activity, , preferably CD8 effector T cell activity, or macrophage activity, preferably CD40-activated macrophage activity.
3. The antibody for use according to claim 1 for use in rendering a cancer susceptible for the treatment with an antibody that binds to human angiopoietin 2 (ANG-2).
4. Use of a combination of
   i) an antibody that binds to human angiopoietin 2 (ANG-2), and
   ii) an agonistic CD40 antibody
      for the manufacture of a medicament
      a) for use in treating or delaying progression of cancer, or b) for use in prolonging the survival of a patient suffering from cancer, or c) for use in stimulating an immune response or function, such as T cell activity (in one embodiment CD8 effector T cell activity) or macrophage activity (in one embodiment CD40-activated macrophage activity), or d) for use in rendering a cancer susceptible for the treatment with an antibody that binds to human angiopoietin 2 (ANG-2).
5. Use of a combination of
   i) an antibody that binds to human angiopoietin 2 (ANG-2), and
   ii) an agonistic CD40 antibody
      for the manufacture of a medicament
      a) for use in treating or delaying progression of cancer, b) for use in prolonging the survival of a patient suffering from cancer.
6. The anti-ANG2 antibody for use or the use according to any one of the preceding embodiments, wherein the anti-ANG2 antibody is human or humanized.
7. The anti-ANG2 antibody for use or the use according to any one of the preceding embodiments, wherein the anti-ANG2 antibody specifically binds to human ANG2 with a K_{D} value of less than 1.0 x 10⁻⁸ mol/l, as determined by surface plasmon resonance (Biacore™).
8. The anti-ANG2 antibody for use or the use according to any one of the preceding embodiments, wherein the anti-ANG2 antibody is an IgG antibody.
9. The anti-ANG2 antibody for use or the use according to any one of the preceding embodiments, wherein the anti-ANG2 antibody inhibits the interaction of human ANG-2 with TIE2 receptor with an IC50 of 15 nM or less.
10. The antibody that binds to human ANG-2 or the use according to any one of the preceding embodiments,
   i) wherein the anti-ANG2 antibody comprises
      (a) a heavy chain variable domain amino acid sequence of SEQ ID NO:1 and a light chain variable domain amino acid sequence of SEQ ID NO:2; or
      (b) a heavy chain variable domain amino acid sequence of SEQ ID NO:3 and a light chain variable domain amino acid sequence of SEQ ID NO:4;
      and
   ii) wherein the agonistic CD40 antibody comprises
      (a) a heavy chain variable domain amino acid sequence of SEQ ID NO: 5 and a light chain variable domain amino acid sequence of SEQ ID NO: 6; or
      (b) a heavy chain variable domain amino acid sequence of SEQ ID NO: 7 and a light chain variable domain amino acid sequence of SEQ ID NO: 8;
11. The antibody that binds to human ANG-2 or the use according to embodiment 10,
   i) wherein the anti-ANG2 antibody comprises
      a heavy chain variable domain amino acid sequence of SEQ ID NO:1 and a light chain variable domain amino acid sequence of SEQ ID NO:2; or
      and
   ii) wherein the agonistic CD40 antibody comprises
   a heavy chain variable domain amino acid sequence of SEQ ID NO: 5 and a light chain variable domain amino acid sequence of SEQ ID NO: 6; or
12. The anti-ANG2 antibody for use or the use according to any one of embodiments 1 to 9, wherein the anti-ANG2 antibody is a bispecific antibody that binds to human ANG-2 and that binds to human VEGF.
13. The anti-ANG2 antibody for use or the use according any one of embodiments 1 to 9, wherein additionally an antibody that binds to human VEGF is administered in combination or is used in the combination with the agonistic CD40 antibody.
14. The antibody that binds to human ANG-2 or the use according to embodiment 12,
   i) wherein the bispecific antibody that binds to human ANG-2 and that binds to human VEGF comprises
      a heavy chain variable domain amino acid sequence of SEQ ID NO:1 and a light chain variable domain amino acid sequence of SEQ ID NO:2; and
      a heavy chain variable domain amino acid sequence of SEQ ID NO:9 and a light chain variable domain amino acid sequence of SEQ ID NO:10;
      and
   ii) wherein the agonistic CD40 antibody comprises
   a heavy chain variable domain amino acid sequence of SEQ ID NO: 5 and a light chain variable domain amino acid sequence of SEQ ID NO: 6.
15. The antibody that binds to human ANG-2 or the use according to embodiment 12,
   i) wherein the bispecific antibody that binds to human ANG-2 and that binds to human VEGF comprises the amino acid sequences of SEQ ID NO: 11, of SEQ ID NO: 12, of SEQ ID NO: 13, and of SEQ ID NO: 14; and
   ii) wherein the agonistic CD40 antibody comprises
   a heavy chain variable domain amino acid sequence of SEQ ID NO: 5 and a light chain variable domain amino acid sequence of SEQ ID NO: 6.
16. The antibody that binds to human ANG-2 or the use according to embodiment 13,
   i) wherein the antibody that binds to human VEGF comprises
      a heavy chain variable domain amino acid sequence of SEQ ID NO:9 and a light chain variable domain amino acid sequence of SEQ ID NO:10;
      and
   ii) wherein the agonistic CD40 antibody comprises
   a heavy chain variable domain amino acid sequence of SEQ ID NO: 5 and a light chain variable domain amino acid sequence of SEQ ID NO: 6.
17. An antibody that binds to human angiopoietin 2 (ANG-2) for use or the use according to any one of the preceding embodiments, wherein the cancer is lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma or lymphocytic leukemia.
18. An antibody that binds to human angiopoietin 2 (ANG-2) for use or the use according to embodiment 17, wherein the cancer comprises a solid tumor.
19. The antibody or use according to embodiments 17 or 18, wherein the cancer is further characterized by ANG-2 expression or overexpression.
20. An antibody that binds to human angiopoietin 2 (ANG-2) for use according to any one of embodiments 17 to 19, wherein the cancer is colon cancer, ovarian cancer, glioblastoma, gastric cancer, pancreatic cancer, breast cancer, lung cancer, hepatocellular cancer.
21. The antibody that binds to human angiopoietin 2 (ANG-2) for use according to any one of the preceding embodiments, wherein in the method of treatment, the anti-ANG2 antibody and the CD40 agonist antibody are administered to the subject simultaneously, separately or sequentially.

### Example 1

### Inhibition of the interaction of human ANG-2 with TIE2 receptor (experiment A)

Blocking of human ANG-2/human Tie2 interaction was shown by receptor interaction ELISA. 384-well Maxisorp plates (Nunc) were coated with 0.5 µg/ml human Tie2 (R&D Systems, UK, Cat.No.313-TI or in house produced material) for 2 h at room temperature and blocked with PBS supplemented with 0.2% Tween-20 and 2% BSA (Roche Diagnostics GmbH, DE) for 1 h at room temperature under shaking. In the meantime, Dilutions of purified antibodies in PBS were incubated together with 0.2 µg/ml huAngiopoietin-1/2 (R&D Systems #923-AN/CF, R&D Systems,UK, Cat.No. 623-AN or in house produced material) for 1 hour at RT. After washing a mixture of 0.5 µg/ml biotinylated anti-Angiopoietin-1/2 clone (R&D Systems #BAF923, BAM0981 R&D Systems,UK) and 1:3000 diluted streptavidin HRP (Roche Diagnostics GmbH, DE, Cat.No.11089153001) was added for 1 h. Thereafter the plates were washed 6 times with PBST. Plates were developed with freshly prepared ABTS reagent (Roche Diagnostics GmbH, DE, buffer #204 530 001, tablets #11 112 422 001) for 30 minutes at RT. Absorbance was measured at 405 nm.

The obtained inhibitory concentrations are summarized in the following table.

| **Antibody** | **ANG2/ Tie2 interaction ELISA** |
|---|---|
| Ang2i-LC06 | 0.1 nM |

### Inhibition of the interaction of human ANG-2 with TIE2 receptor (experiment B)

The interaction ELISA was performed on 384 well microtiter plates (MicroCoat, DE, Cat.No. 464718) at RT. After each incubation step plates were washed 3 times with PBST. ELISA plates were coated with 5 µg/ml Tie-2 protein for 1 hour (h). Thereafter the wells were blocked with PBS supplemented with 0.2% Tween-20 and 2% BSA (Roche Diagnostics GmbH, DE) for 1 h. Dilutions of purified bispecific Xmab antibodies in PBS were incubated together with 0.2 µg/ml huAngiopoietin-2 (R&D Systems, UK, Cat.No. 623-AN) for 1 h at RT. After washing a mixture of 0.5 µg/ml biotinylated anti-Angiopoietin-2 clone BAM0981 (R&D Systems, UK) and 1:3000 diluted streptavidin HRP (Roche Diagnostics GmbH, DE, Cat.No. 11089153001) was added for 1 h. Thereafter the plates were washed 3 times with PBST. Plates are developed with freshly prepared ABTS reagent (Roche Diagnostics GmbH, DE, buffer #204 530 001, tablets #11 112 422 001) for 30 minutes at RT. Absorbance was measured at 405 nm and the IC50 was determined.

XMab1, a bispecific antibody that binds to human ANG2 and to human VEGF (see WO2011/117329 and sequences SEQ ID NOs:11- 14, showed an inhibition of ANG-2 binding to Tie-2 (ANG2/Tie2 receptor interaction inhibition) with an IC50 of 12 nM.

### Example 2

### In vivo anti-tumor efficacy of Anti-ANG2 antibodies in combination with an agonistic CD40 antibody (delaying of progression (tumor growth) of cancer and prolonging the survival of patients treated)

### Methods

C57BL/6 mice were s.c. injected with 500.000 syngeneic MC-38 tumor cells and treated beginning day 16 when tumors reached a size of 40-60 mm³.

Mice were euthanized according to veterinary regulations when tumors reached a size of 1500 mm³ (endpoint). As anti-ANG2 antibody a monospecific IgG1 antibody based on the VH and VL of <ANG-2> Ang2i_LC06 (SEQ ID NOs:3-4) was used. As mouse-cross reactive surrogate VEGF antibody based on the VH and VL of <VEGF> B20-4.1 (SEQ ID NOs:17-18) was used instead of the VH and VL of <VEGF> bevacizumab (SEQ ID NOs:9-10), as bevacizumab is not mouse crossreactive . These antibodies were used either alone or in combination. For similar reasons instead of the agonistic CD40 antibody CP-870,893 (antibody 21.4.1 of U.S.7,338,660) (see VH and VL of SEQ ID NOs:5-6) the mouse cross-reactive agonistic CD40 antibody Clone 1C10 (see Santos-Argumedo L. et al., Cell Immunol. 156 (1994) 272-285, Heath AW et al. Eur J Immunol 24 (1994) 1828-34; obtainable e.g. from Abnova Catalog #MAB5607) was used, specifically in aa mouse IgG1 format (which can be either generated from the variable regions VH and VL of Clone 1C10 in combination with a mouse IgG1 constant region or which is also obtainable from Rockefeller University). For comparison also the combination with monospecific anti-VEGF antibody <VEGF> B20-4.1 (SEQ ID NOs:17-18) was examined. However similar experiments with transgenic humanized mice or clinical trials can be conducted using agonistic CD40 antibody CP-870,893 (antibody 21.4.1 of U.S.7,338,660) and the bispecific ANG2/VEGF antibody XMab1 ((SEQ ID NOs:11-14) expecting similar result based on the mechanism of action.

Treatments were applied according to the following schedule below at the indicated doses.

| Tumor cell injection | Starting at day 500.000 syngeneic MC-38 tumor cells injected and treatment started at day 16 when tumors reached a size of 40-60 mm³ |
|---|---|
| Agonistic CD40 antibody alone | 5 mg/kg i.p. at day 16, 18, 21 and 24 |
| Anti-ANG2 antibody alone | 10 mg/kg i.p. at day 16 and 21 |
| Anti-VEGF antibody alone | 10 mg/kg i.p at day 16 and 21 |
| anti-ANG2 antibody with anti-VEGF antibody (Anti-VEGF/Ang-2) | both at a dose of 10 mg/kg i.p at day 16 and 21 |
| Anti-ANG2 antibody | 10 mg/kg i.p. at day 16 and 21 |
| with | |
| agonistic CD40 antibody | 5 mg/kg i.p. at day 16 , 18, 21 and 24 |
| anti-ANG2 antibody with anti-VEGF antibody (Anti-VEGF/Ang-2) | both at a dose of 10 mg/kg i.p. at day 16 and 21 |
| with | |
| agonistic CD40 antibody | 5 mg/kg i.p. at day 16 , 18, 21 and 24 |
| Anti-VEGF antibody | 10 mg/kg i.p. at day 16 and 21 |
| with | |
| agonistic CD40 antibody | 5 mg/kg i.p. at day 16 , 18, 21 and 24 |

### Results

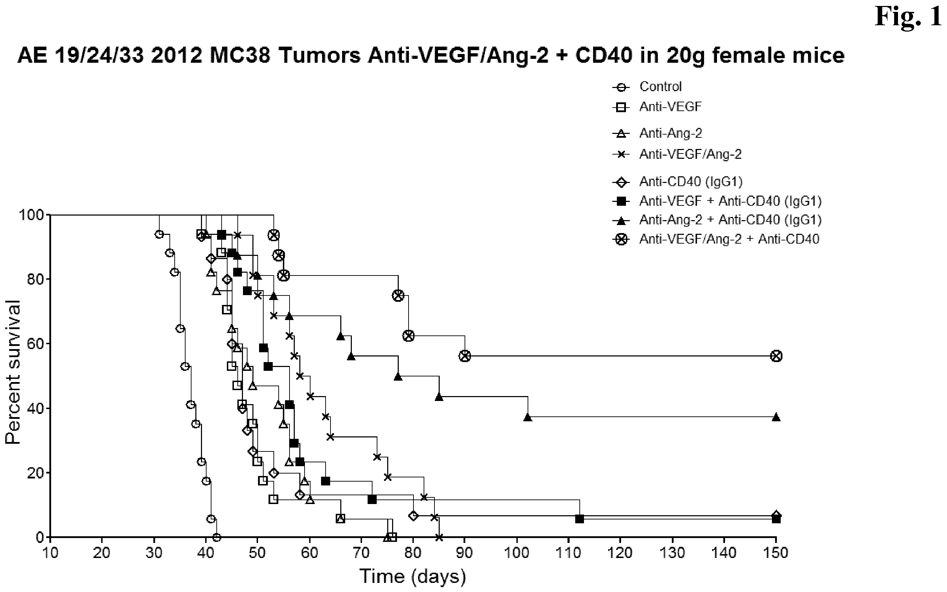

Results are shown in Figure 1. The graph represents pooled data from 3 independent experiments. Mice were treated with the antibody combinations indicated in the figure.

### Summary

Our data demonstrate that all used reagents do display some anti-tumor activity on their own. While the combination of an agonistic anti-CD40 antibody did only display small effects when combined with anti-VEGF antibody, the combination of agonistic CD40 antibody with anti-ANG2 antibodies (either monospecific ANG2 antibodies or the combination of anti-ANG2/ anti-VEGF antibodies showed a strong synergistic effect on tumor cell growth, the delay of progression and the prolongation of survival. The strongest effect could be observed for the combination of agonistic CD40 antibody and the combination of anti-ANG2/ anti-VEGF antibodies. A large proportion of tumor bearing mice were cured by the combined treatment with either anti-Ang-2 and anti-CD40 or the combination anti-ANG2/ anti-VEGF antibodies (anti-VEGF/Ang-2) and anti-CD40. These combinations may therefore provide substantial therapeutic benefits for cancer patients.

### Example 3

### In vivo anti-tumor efficacy of Anti-ANG2 antibodies (bispecific) in combination with an agonistic CD40 antibody in subcutaneous syngeneic MC38 colon carcinoma model

Cells of the murine colorectal adenocarcinoma cell line MC-38 (obtained from Beckman Research Institute of the City of Hope, California, USA) were cultured in Dulbecco's Modified Eagle Medium (DMEM, PAN Biotech) supplemented with 10% FCS and 2mM L-glutamine at 37°C in a water saturated atmosphere at 5% CO2. At the day of inoculation, MC38 tumor cells were harvested with PBS from culture flasks and transferred into culture medium, centrifuged, washed once and re-suspended in PBS. For injection of cells, the final titer was adjusted to 1×107 cells/ml. Subsequently 100 µl of this suspension (1×106 cells) were inoculated subcutaneously into 6-10 weeks old female C57BL/6N mice. Groups of animals were treated with control antibodies (MOPC-21 (10 mg/kg i.p. once weekly) and/or 2A3 (100 µg i.p. once); Bio X Cell, West Lebanon), and with a bispecific anti-ANG2 antibody in combination with anti-CD40 monoclonal antibody FGK45 (agonist CD40 rat anti-mouse IgG2a mAb FGK45 (S. P. Schoenberger, et al, Nature, 393, 480 (1998), available from BioXcell) CD40 clone FGK.45, 100 µg, i.p., once simultaneously with first dose of bispecific ANG2/VEGF antibody). the bispecific was a bispecific ANG2/VEGF antibody based on the VH and VL of <ANG-2> E6Q (SEQ ID NOs:1-2) for the ANG2 binding arm. As mouse-cross reactive surrogate VEGF binding arm the VH and VL of <VEGF> B20-4.1 (SEQ ID NOs:17-18) for the bispecific antibody were used instead of the VH and VL of <VEGF> bevacizumab (SEQ ID NOs:9-10). The structure , however was the same as that for XMab1 (only VH/VLs <VEGF> were exchanged for the mouse cross-reactive ones of B20-4.1)).

Treatment started after tumors were established and had reached an average size of 50 to 80 mm3 for monotherapy or 200mm3 for combination. Tumor volume was measured twice a week and animal weights were monitored in parallel. Results are shown in Figure 7. Combination of bispecific ANG2/VEGF antibody with agonistic CD40 mAb shows improved anti-tumor efficacy over monotherapies in syngenic MC38 mouse adenocarcinoma model with tumors going in regression at day 21 (with TGI >100%). At day 32, 5 out of 10 animals were tumor free.

| | TGI | Study |
|---|---|---|
| Agonistic CD40 antibody | 62% (day 21) | CSF1R_Pz_MC38_008 |
| Bispecific Ang2/ VEGF antibody | 30% (day 20) | CSF1R_Pz_MC38_009 |
| Combination of Agonistic CD40 antibody and Bispecific Ang2/ VEGF antibody | >100% (day 21) | CSF1R_Pz_MC38_011 |

### Example 4

### Anti-tumor efficacy of the Ang2/VEGF antibody (CrossMab; LC06/B20.4.1) and the CD40 antibody (FGK4.5; iTME-0004-0005) alone or in combination in the syngeneic CT26WT model in female Balb/c mice

### Experimental Procedures

### Test agents

The bispecific ANG2/VEGF antibody based on the VH and VL of <ANG-2> E6Q (SEQ ID NOs:1-2) for the ANG2 binding arm. As mouse-cross reactive surrogate VEGF binding arm the VH and VL of <VEGF> B20-4.1 (SEQ ID NOs:17-18) for the bispecific antibody were used instead of the VH and VL of <VEGF> bevacizumab (SEQ ID NOs:9-10). The structure , however was the same as that for XMab1 (only VH/VLs <VEGF> were exchanged for the mouse cross-reactive ones of B20-4.1)). The bispecific ANG2/VEGF antibody was generated at Roche Diagnostics GmbH, Penzberg, Germany. The CD40 antibody (FGK4.5; iTME-0004-0005) was obtained from Adipogen/Biomol.

Antibody buffer included 20 mM histidine and 140 mM sodium chloride (pH 6.0). Antibody solutions were diluted appropriately in the above mentioned buffer from stock prior to administrations.

### Cell lines and culture conditions

The murine CT26WT cell line was routinely cultured in RPMI 1640 supplemented with 10% fetal bovine serum (PAA Laboratories, Austria) and 2 mM L-glutamine at 37°C in a water-saturated atmosphere at 5% CO2.

### Animals

Female Balb/c mice aged 6-7 weeks at arrival (purchased from Charles River, Sulzfeld, Germany) were maintained under specific-pathogen-free condition with daily cycles of 12 h light /12 h darkness according to committed guidelines (GV-Solas; Felasa; TierschG). Experimental study protocol was reviewed and approved by local government (Regierung von Oberbayern; registration no. 55.2-1-54-2531.2-32-10). After arrival animals were maintained in the animal facility for one week to get accustomed to new environment and for observation. Continuous health monitoring was carried out on regular basis. Diet food (Altromin) and water (filtered) were provided ad libitum.

### Monitoring

Animals were controlled daily for clinical symptoms and detection of adverse effects. For monitoring throughout the experiment body weight of animals was documented two times weekly and tumor volume was measured by caliper after randomization.

### Treatment of animals

Animal treatment was started at the day of randomization at a tumor volume of about 130 mm³ 12 days after tumor cell inoculation. Following dosages, route of administration and treatment schedules have been applied:

**Table 1: Applied dosages, route of administration and treatment schedules**

| **Compound** | **Dose (mg/kg)** | **Route of administration** | **Treatment schedule** |
|---|---|---|---|
| Histidin buffer | --- | IP | once weekly x5 |
| RO6872840 Crossmab (LC06/B20.4.1) | 10 | IP | once weekly x2 (Day 12/19) |
| CD40 (FGK4.5 iTME-0004-0005) | 100µg/mouse | IP | Day 12 |
| CD40 | 100µg/mouse | IP | Day 12 |
| + | + | | + |
| LC06/B20.4.1 | 10 | | once weekly x2 (Day 12/19) |

## Claims

1. An antibody that binds to human angiopoietin 2 (ANG-2) a) for use in a method of treating or delaying progression of cancer, or b) for use in a method of prolonging the survival of a patient suffering from cancer, wherein the method comprises administration of said antibody in combination with an agonistic CD40 antibody.

2. The antibody for use according to claim 1 for use in stimulating an immune response or function, such as T cell activity, , preferably CD8 effector T cell activity, or macrophage activity, preferably CD40-activated macrophage activity.

3. The antibody for use according to claim 1 for use in rendering a cancer susceptible for the treatment with an antibody that binds to human angiopoietin 2 (ANG-2).

4. The anti-ANG2 antibody for use according to any one of the preceding claims, wherein the anti-ANG2 antibody is human or humanized.

5. The anti-ANG2 antibody for use according to any one of the preceding claims, wherein the anti-ANG2 antibody specifically binds to human ANG2 with a K_{D} value of less than 1.0 x 10⁻⁸ mol/l, as determined by surface plasmon resonance (Biacore™).

6. The anti-ANG2 antibody for use according to any one of the preceding claims, wherein the anti-ANG2 antibody is an IgG antibody.

7. The anti-ANG2 antibody for use according to any one of the preceding claims, wherein the anti-ANG2 antibody inhibits the interaction of human ANG-2 with TIE2 receptor with an IC50 of 15 nM or less.

8. The anti-ANG2 antibody for use according to any one of the preceding claims,
i) wherein the anti-ANG2 antibody comprises
(a) a heavy chain variable domain amino acid sequence of SEQ ID NO:1 and a light chain variable domain amino acid sequence of SEQ ID NO:2; or
(b) a heavy chain variable domain amino acid sequence of SEQ ID NO:3 and a light chain variable domain amino acid sequence of SEQ ID NO:4;
and
ii) wherein the agonistic CD40 antibody comprises
(a) a heavy chain variable domain amino acid sequence of SEQ ID NO: 5 and a light chain variable domain amino acid sequence of SEQ ID NO: 6; or
(b) a heavy chain variable domain amino acid sequence of SEQ ID NO: 7 and a light chain variable domain amino acid sequence of SEQ ID NO: 8.

9. The anti-ANG2 antibody for use according to claim 8,
i) wherein the anti-ANG2 antibody comprises
a heavy chain variable domain amino acid sequence of SEQ ID NO:1 and a light chain variable domain amino acid sequence of SEQ ID NO:2;
and
ii) wherein the agonistic CD40 antibody comprises
a heavy chain variable domain amino acid sequence of SEQ ID NO: 5 and a light chain variable domain amino acid sequence of SEQ ID NO: 6.

10. The anti-ANG2 antibody for use according to any one of claims 1 to 7 , wherein the anti-ANG2 antibody is a bispecific antibody that binds to human ANG-2 and that binds to human VEGF.

11. The anti-ANG2 antibody for use according to claim 10,
i) wherein the bispecific antibody that binds to human ANG-2 and that binds to human VEGF comprises
a heavy chain variable domain amino acid sequence of SEQ ID NO:1 and a light chain variable domain amino acid sequence of SEQ ID NO:2; and
a heavy chain variable domain amino acid sequence of SEQ ID NO:9 and a light chain variable domain amino acid sequence of SEQ ID NO:10;
and
ii) wherein the agonistic CD40 antibody comprises
a heavy chain variable domain amino acid sequence of SEQ ID NO: 5 and a light chain variable domain amino acid sequence of SEQ ID NO: 6.

12. The anti-ANG2 antibody for use ANG-2 according to 10,
i) wherein the bispecific antibody that binds to human ANG-2 and that binds to human VEGF comprises the amino acid sequences of SEQ ID NO: 11, of SEQ ID NO: 12, of SEQ ID NO: 13, and of SEQ ID NO: 14; and
ii) wherein the agonistic CD40 antibody comprises
a heavy chain variable domain amino acid sequence of SEQ ID NO: 5 and a light chain variable domain amino acid sequence of SEQ ID NO: 6.

13. The antibody that binds to human angiopoietin 2 (ANG-2) for use according to any one of the preceding claims, wherein the cancer is lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma or lymphocytic leukemia.

14. The antibody that binds to human angiopoietin 2 (ANG-2) for use according to claim 13, wherein the cancer comprises a solid tumor.

15. The antibody that binds to human angiopoietin 2 (ANG-2) for use according to any one of the preceding claims, wherein in the the anti-ANG2 antibody and the agonistic CD40 antibody are administered simultaneously.

16. The antibody that binds to human angiopoietin 2 (ANG-2) for use according to any one of the preceding claims, wherein in the the anti-ANG2 antibody and the agonistic CD40 antibody are administered sequentially.

## Patentansprüche

1. Antikörper, der an Human-Angiopoetin 2 (ANG2) bindet a) zur Verwendung in einem Verfahren zum Behandeln oder Verzögern des Fortschreitens von Krebs oder b) zur Verwendung in einem Verfahren zum Verlängern des Überlebens eines an Krebs leidenden Patienten, wobei das Verfahren die Verabreichung des Antikörpers in Kombination mit einem agonistischen CD40-Antikörper umfasst.

2. Antikörper zur Verwendung nach Anspruch 1 zur Verwendung dahingehend, eine Immunantwort oder -funktion, wie etwa eine T-Zell-Aktivität, bevorzugt CD8-Effektor-T-Zell-Aktivität oder Makrophagenaktivität, bevorzugt CD40-aktivierte Makrophagenaktivität, zu stimulieren.

3. Antikörper zur Verwendung nach Anspruch 1 zur Verwendung dahingehend, einen Krebs empfänglich für die Behandlung mit einem Antikörper, der an Human-Angiopoetin 2 (ANG2) bindet, zu machen.

4. Anti-ANG2-Antikörper zur Verwendung nach einem der vorhergehenden Ansprüche, wobei der Anti-ANG2-Antikörper human oder humanisiert ist.

5. Anti-ANG2-Antikörper zur Verwendung nach einem der vorhergehenden Ansprüche, wobei der Anti-ANG2-Antikörper spezifisch an Human-ANG2 mit einem K_{D}-Wert von weniger als 1,0 x 10⁻⁸ mol/l bindet, wie anhand von Oberflächenplasmonenresonanz ermittelt (Biacore™).

6. Anti-ANG2-Antikörper zur Verwendung nach einem der vorhergehenden Ansprüche, wobei der Anti-ANG2-Antikörper ein IgG-Antikörper ist.

7. Anti-ANG2-Antikörper zur Verwendung nach einem der vorhergehenden Ansprüche, wobei der Anti-ANG2-Antikörper die Interaktion von Human-ANG2 mit dem TIE2-Rezeptor mit einer IC50 von 15 nM oder weniger hemmt.

8. Anti-ANG2-Antikörper zur Verwendung nach einem der vorhergehenden Ansprüche,
i) wobei der Anti-ANG2-Antikörper Folgendes umfasst:
(a) eine Aminosäuresequenz von SEQ ID NO: 1 der variablen Domäne der schweren Kette und eine Aminosäuresequenz von SEQ ID NO: 2 der variablen Domäne der leichten Kette; oder
(b) eine Aminosäuresequenz von SEQ ID NO: 3 der variablen Domäne der schweren Kette und eine Aminosäuresequenz von SEQ ID NO: 4 der variablen Domäne der leichten Kette;
und
ii) wobei der agonistische CD40-Antikörper Folgendes umfasst:
(a) eine Aminosäuresequenz von SEQ ID NO: 5 der variablen Domäne der schweren Kette und eine Aminosäuresequenz von SEQ ID NO: 6 der variablen Domäne der leichten Kette; oder
(b) eine Aminosäuresequenz von SEQ ID NO: 7 der variablen Domäne der schweren Kette und eine Aminosäuresequenz von SEQ ID NO: 8 der variablen Domäne der leichten Kette.

9. Anti-ANG2-Antikörper zur Verwendung nach Anspruch 8,
i) wobei der Anti-ANG2-Antikörper Folgendes umfasst:
eine Aminosäuresequenz von SEQ ID NO: 1 der variablen Domäne der schweren Kette und eine Aminosäuresequenz von SEQ ID NO: 2 der variablen Domäne der leichten Kette;
und
ii) wobei der agonistische CD40-Antikörper Folgendes umfasst:
eine Aminosäuresequenz von SEQ ID NO: 5 der variablen Domäne der schweren Kette und eine Aminosäuresequenz von SEQ ID NO: 6 der variablen Domäne der leichten Kette.

10. Anti-ANG2-Antikörper zur Verwendung nach einem der Ansprüche 1 bis 7, wobei der Anti-ANG2-Antikörper ein bispezifischer Antikörper ist, der an Human-ANG2 bindet und der an den humanen VEGF bindet.

11. Anti-ANG2-Antikörper zur Verwendung nach Anspruch 10,
i) wobei der bispezifischer Antikörper, der an Human-ANG2 bindet und der an den humanen VEGF bindet, Folgendes umfasst:
eine Aminosäuresequenz von SEQ ID NO: 1 der variablen Domäne der schweren Kette und eine Aminosäuresequenz von SEQ ID NO: 2 der variablen Domäne der leichten Kette; und
eine Aminosäuresequenz von SEQ ID NO: 9 der variablen Domäne der schweren Kette und eine Aminosäuresequenz von SEQ ID NO: 10 der variablen Domäne der leichten Kette;
und
ii) wobei der agonistische CD40-Antikörper Folgendes umfasst:
eine Aminosäuresequenz von SEQ ID NO: 5 der variablen Domäne der schweren Kette und eine Aminosäuresequenz von SEQ ID NO: 6 der variablen Domäne der leichten Kette.

12. Anti-ANG2-Antikörper zur Verwendung ANG2 nach 10,
i) wobei der bispezifischer Antikörper, der an Human-ANG2 bindet und der an den humanen VEGF bindet, die Aminosäuresequenzen von SEQ ID NO: 11, von SEQ ID NO: 12, von SEQ ID NO: 13 und von SEQ ID NO: 14 umfasst; und
ii) wobei der agonistische CD40-Antikörper Folgendes umfasst:
eine Aminosäuresequenz von SEQ ID NO: 5 der variablen Domäne der schweren Kette und eine Aminosäuresequenz von SEQ ID NO: 6 der variablen Domäne der leichten Kette.

13. Antikörper, der an Human-Angiopoetin 2 (ANG2) bindet, zur Verwendung nach einem der vorhergehenden Ansprüche, wobei es sich bei dem Krebs um Lungenkrebs, nichtkleinzelligen Lungenkrebs (NSCL), bronchioalveolarzelligen Lungenkrebs, Knochenkrebs, Bauchspeicheldrüsenkrebs, Hautkrebs, Krebs im Kopf- oder Nackenbereich, kutanes oder intraokulares Melanom, Gebärmutterkrebs, Eierstockkrebs, Mastdarmkrebs, Krebs der Analregion, Magenkrebs, Dickdarmkrebs, Brustkrebs, Gebärmutterkrebs, Tubenkarzinom, Endometriumkarzinom, Zervixkarzinom, Vaginalkarzinom, Vulvakarzinom, Hodgkin-Lymphom, Speiseröhrenkrebs, Dünndarmkrebs, Krebs des endokrinen Systems, Schilddrüsenkrebs, Krebs der Nebenschilddrüse, Nebennierenkrebs, Weichgewebesarkom, Harnröhrenkrebs, Peniskrebs, Prostatakrebs, Blasenkrebs, Nieren- oder Harnleiterkrebs, Nierenzellkarzinom, Nierenbeckenkarzinom, Mesotheliom, hepatozelluläres Karzinom, Gallenkrebs, Neoplasmen des zentralen Nervensystems (ZNS), Wirbelsäulentumoren, Hirnstammgliom, Glioblastoma multiforme, Astrozytome, Schwannome, Ependymone, Medulloblastome, Meningiome, Plattenepithelkarzinome, Hypophysenadenom, Lymphom oder lymphozytische Leukämie handelt.

14. Antikörper, der an Human-Angiopoetin 2 (ANG2) bindet, zur Verwendung nach Anspruch 13, wobei der Krebs einen soliden Tumor umfasst.

15. Antikörper, der an Human-Angiopoetin 2 (ANG2) bindet, zur Verwendung nach einem der vorhergehenden Ansprüche, wobei der Anti-ANG2-Antikörper und der agonistische CD40-Antikörper zeitgleich verabreicht werden.

16. Antikörper, der an Human-Angiopoetin 2 (ANG2) bindet, zur Verwendung nach einem der vorhergehenden Ansprüche, wobei der Anti-ANG2-Antikörper und der agonistische CD40-Antikörper nacheinander verabreicht werden.

## Revendications

1. Anticorps se liant à l'angiopoïétine 2 (ANG-2) humaine
a) destiné à être utilisé dans une méthode de traitement ou de ralentissement de la progression du cancer, ou b) destiné à être utilisé dans une méthode de prolongement de la survie d'un patient souffrant du cancer, la méthode comprenant l'administration dudit anticorps en association avec un anticorps agoniste de CD40.

2. Anticorps destiné à être utilisé selon la revendication 1, à utiliser dans la stimulation d'une réponse ou d'une fonction immunitaire, telle que l'activité des lymphocytes T, de préférence l'activité des lymphocytes T effecteurs CD8, ou l'activité des macrophages, de préférence l'activité des macrophages activés par CD40.

3. Anticorps destiné à être utilisé selon la revendication 1, à utiliser pour rendre un cancer sensible au traitement avec un anticorps se liant à l'angiopoïétine 2 (ANG-2) humaine.

4. Anticorps anti-ANG2 destiné à être utilisé selon l'une quelconque des revendications précédentes, dans lequel l'anticorps anti-ANG2 est humain ou humanisé.

5. Anticorps anti-ANG2 destiné à être utilisé selon l'une quelconque des revendications précédentes, dans lequel l'anticorps anti-ANG2 se lie spécifiquement à l'ANG2 humaine avec une valeur de K_{D} inférieure à 1,0 x 10⁻⁸ mol/l, telle que déterminée par résonance plasmonique de surface (Biacore™).

6. Anticorps anti-ANG2 destiné à être utilisé selon l'une quelconque des revendications précédentes, dans lequel l'anticorps anti-ANG2 est un anticorps IgG.

7. Anticorps anti-ANG2 destiné à être utilisé selon l'une quelconque des revendications précédentes, dans lequel l'anticorps anti-ANG2 inhibe l'interaction de l'ANG-2 humaine avec le récepteur TIE2 avec une CI50 inférieure ou égale à 15 nM.

8. Anticorps anti-ANG2 destiné à être utilisé selon l'une quelconque des revendications précédentes,
i) dans lequel l'anticorps anti-ANG2 comprend
(a) une séquence d'acides aminés de domaine variable de chaîne lourde de SEQ ID NO: 1 et une séquence d'acides aminés de domaine variable de chaîne légère de SEQ ID NO: 2 ; ou
(b) une séquence d'acides aminés de domaine variable de chaîne lourde de SEQ ID NO: 3 et une séquence d'acides aminés de domaine variable de chaîne légère de SEQ ID NO: 4 ;
et
ii) dans lequel l'anticorps agoniste de CD40 comprend
(a) une séquence d'acides aminés de domaine variable de chaîne lourde de SEQ ID NO: 5 et une séquence d'acides aminés de domaine variable de chaîne légère de SEQ ID NO: 6 ; ou
(b) une séquence d'acides aminés de domaine variable de chaîne lourde de SEQ ID NO: 7 et une séquence d'acides aminés de domaine variable de chaîne légère de SEQ ID NO: 8.

9. Anticorps anti-ANG2 destiné à être utilisé selon la revendication 8,
i) dans lequel l'anticorps anti-ANG2 comprend
une séquence d'acides aminés de domaine variable de chaîne lourde de SEQ ID NO: 1 et une séquence d'acides aminés de domaine variable de chaîne légère de SEQ ID NO: 2 ;
et
ii) dans lequel l'anticorps agoniste de CD40 comprend
une séquence d'acides aminés de domaine variable de chaîne lourde de SEQ ID NO: 5 et une séquence d'acides aminés de domaine variable de chaîne légère de SEQ ID NO: 6.

10. Anticorps anti-ANG2 destiné à être utilisé selon l'une quelconque des revendications 1 à 7, dans lequel l'anticorps anti-ANG2 est un anticorps bispécifique se liant à l'ANG-2 humaine et se liant au VEGF humain.

11. Anticorps anti-ANG2 destiné à être utilisé selon la revendication 10,
i) dans lequel l'anticorps bispécifique se liant à l'ANG-2 humaine et se liant au VEGF humain comprend
une séquence d'acides aminés de domaine variable de chaîne lourde de SEQ ID NO: 1 et une séquence d'acides aminés de domaine variable de chaîne légère de SEQ ID NO: 2 ; et
une séquence d'acides aminés de domaine variable de chaîne lourde de SEQ ID NO: 9 et une séquence d'acides aminés de domaine variable de chaîne légère de SEQ ID NO: 10 ;
et
ii) dans lequel l'anticorps agoniste de CD40 comprend
une séquence d'acides aminés de domaine variable de chaîne lourde de SEQ ID NO: 5 et une séquence d'acides aminés de domaine variable de chaîne légère de SEQ ID NO: 6.

12. Anticorps anti-ANG2 destiné à être utilisé ANG-2 selon la revendication 10,
i) dans lequel l'anticorps bispécifique se liant à l'ANG-2 humaine et se liant au VEGF humain comprend les séquences d'acides aminés de SEQ ID NO: 11, de SEQ ID NO: 12, de SEQ ID NO: 13, et de SEQ ID NO: 14 ; et
ii) dans lequel l'anticorps agoniste de CD40 comprend
une séquence d'acides aminés de domaine variable de chaîne lourde de SEQ ID NO: 5 et une séquence d'acides aminés de domaine variable de chaîne légère de SEQ ID NO: 6.

13. Anticorps se liant à l'angiopoïétine 2 (ANG-2) humaine destiné à être utilisé selon l'une quelconque des revendications précédentes, dans lequel le cancer est un cancer bronchopulmonaire, un cancer bronchopulmonaire non à petites cellules (CBNPC), un cancer du poumon bronchiolo-alvéolaire, un cancer des os, un cancer du pancréas, un cancer de la peau, un cancer de la tête ou du cou, un mélanome cutané ou intraoculaire, un cancer de l'utérus, un cancer de l'ovaire, un cancer du rectum, un cancer de la région anale, un cancer de l'estomac, un cancer gastrique, un cancer du côlon, un cancer du sein, un cancer de l'utérus, un carcinome des trompes de Fallope, un carcinome de l'endomètre, un carcinome du col de l'utérus, un carcinome du vagin, un carcinome de la vulve, la maladie de Hodgkin, un cancer de l'oesophage, un cancer de l'intestin grêle, un cancer du système endocrinien, un cancer de la glande thyroïde, un cancer de la glande parathyroïde, un cancer de la glande surrénale, un sarcome des tissus mous, un cancer de l'urètre, un cancer du pénis, un cancer de la prostate, un cancer de la vessie, un cancer des reins ou de l'uretère, un carcinome des cellules rénales, un carcinome du bassinet du rein, un mésothéliome, un cancer hépatocellulaire, un cancer biliaire, des néoplasmes du système nerveux central (SNC), des tumeurs de l'axe spinal, des gliomes du tronc cérébral, un glioblastome multiforme, des astrocytomes, des schwannomes, des épendymomes, des médulloblastomes, des méningiomes, des carcinomes à cellules squameuses, un adénome pituitaire, un lymphome ou une leucémie lymphoïde.

14. Anticorps se liant à l'angiopoïétine 2 (ANG-2) humaine destiné à être utilisé selon la revendication 13, dans lequel le cancer comprend une tumeur solide.

15. Anticorps se liant à l'angiopoïétine 2 (ANG-2) humaine destiné à être utilisé selon l'une quelconque des revendications précédentes, dans lequel l'anticorps anti-ANG2 et l'anticorps agoniste de CD40 sont administrés simultanément.

16. Anticorps se liant à l'angiopoïétine 2 (ANG-2) humaine destiné à être utilisé selon l'une quelconque des revendications précédentes, dans lequel l'anticorps anti-ANG2 et l'anticorps agoniste de CD40 sont administrés séquentiellement.


### SEQUENCE LISTING

<110> F. Hoffmann-La Roche AG
<120> Combination therapy with an anti-ANG2 antibody and a CD40 agonist
<130> P31903 WO
<150> EP13198753.9
   <151> 2013-12-20
<150> EP14158331.0
   <151> 2014-03-07
<160> 18
<170> PatentIn version 3.5
<210> 1
   <211> 128
   <212> PRT
   <213> Artificial
<220>
   <223> variable heavy chain domain VH of <ANG-2> E6Q
<400> 1
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
<210> 2
   <211> 108
   <212> PRT
   <213> Artificial
<220>
   <223> variable light chain domain VL of < ANG-2> E6Q
<400> 2
   Gln Pro Gly Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Tyr Val Phe Gly Thr Gly Thr Lys Val Thr Val Leu
               100                 105
<210> 3
   <211> 129
   <212> PRT
   <213> Artificial
<220>
   <223> variable heavy chain domain VH of <ANG-2> Ang2i_LC06
<400> 3
   Gln Val Gln Leu Val Glu Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser
<210> 4
   <211> 110
   <212> PRT
   <213> Artificial
<220>
   <223> variable light chain domain VL of < ANG-2> Ang2i_LC06
<400> 4
   Gln Pro Gly Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Tyr Val Phe Gly Thr Gly Thr Lys Val Thr Val Leu Gly Gln
               100                 105                 110
<210> 5
   <211> 126
   <212> PRT
   <213> Homo sapiens
<400> 5
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asp Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Asn Arg Leu Arg Ser Asp Asp Thr Ala Val Tyr Tyr Cys
                   85                  90                  95
   Ala Arg Asp Gln Pro Leu Gly Tyr Cys Thr Asn Gly Val Cys Ser Tyr
               100                 105                 110
   Phe Asp Tyr Trp Gly Gln Gly Thr Leu Val Thr Val Ser Ser
           115                 120                 125
<210> 6
   <211> 107
   <212> PRT
   <213> Homo sapiens
<400> 6
   Asp Ile Gln Met Thr Gln Ser Pro Ser Ser Val Ser Ala Ser Val Gly
   1               5                   10                  15
   Asp Arg Val Thr Ile Thr Cys Arg Ala Ser Gln Gly Ile Tyr Ser Trp
               20                  25                  30
   Leu Ala Trp Tyr Gln Gln Lys Pro Gly Lys Ala Pro Asn Leu Leu Ile
           35                  40                  45
   Tyr Thr Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ala Asn Ile Phe Pro Leu
                   85                  90                  95
   Thr Phe Gly Gly Gly Thr Lys Val Glu Ile Lys
   100 105
<210> 7
   <211> 113
   <212> PRT
   <213> Artificial
<220>
   <223> humanized S2C6 heavy chain variabel domain variant
<400> 7
   Glu Val Gln Leu Val Glu Ser Gly Gly Gly Leu Val Gln Pro Gly Gly
   1 5 10 15
   Ser Leu Arg Leu Ser Cys Ala Ala Ser Gly Tyr Ser Phe Thr Gly Tyr
   20 25 30
   Tyr Ile His Trp Val Arg Gln Ala Pro Gly Lys Gly Leu Glu Trp Val
   35 40 45
   Ala Arg Val Ile Pro Asn Ala Gly Gly Thr Ser Tyr Asn Gln Lys Phe
   50 55 60
   Lys Gly Arg Phe Thr Leu Ser Val Asp Asn Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Glu Gly Ile Tyr Trp Trp Gly Gln Gly Thr Leu Val Thr Val
   100 105 110
   Ser
<210> 8
   <211> 113
   <212> PRT
   <213> Artificial
<220>
   <223> humanized S2C6 light chain variabel domain variant
<400> 8
   Asp Ile Gln Met Thr Gln Ser Pro Ser Ser Leu Ser Ala Ser Val Gly
   1 5 10 15
   Asp Arg Val Thr Ile Thr Cys Arg Ser Ser Gln Ser Leu Val His Ser
   20 25 30
   Asn Gly Asn Thr Phe Leu His Trp Tyr Gln Gln Lys Pro Gly Lys Ala
   35 40 45
   Pro Lys Leu Leu Ile Tyr Thr Val Ser Asn Arg Phe Ser Gly Val Pro
   50 55 60
   Ser Arg Phe Ser Gly Ser Gly Ser Gly Thr Asp Phe Thr Leu Thr Ile
   65 70 75 80
   Ser Ser Leu Gln Pro Glu Asp Phe Ala Thr Tyr Phe Cys Ser Gln Thr
   85 90 95
   Thr His Val Pro Trp Thr Phe Gly Gln Gly Thr Lys Val Glu Ile Lys
   100 105 110
   Arg
<210> 9
   <211> 123
   <212> PRT
   <213> Artificial
<220>
   <223> variable heavy chain domain VH of <VEGF> bevacizumab
<400> 9
<210> 10
   <211> 107
   <212> PRT
   <213> Artificial
<220>
   <223> variable light chain domain VL of <VEGF> bevacizumab
<400> 10
<210> 11
   <211> 214
   <212> PRT
   <213> Artificial
<220>
   <223> XMab1 -<VEGF> light chain
<400> 11
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 12
   <211> 213
   <212> PRT
   <213> Artificial
<220>
   <223> XMab1 -<ANG2> light chain
<400> 12
   Gln Pro Gly Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Tyr Val Phe Gly Thr Gly Thr Lys Val Thr Val Leu Ser Ser Ala Ser
               100                 105                 110
   Thr Lys Gly Pro Ser Val Phe Pro Leu Ala Pro Ser Ser Lys Ser Thr
           115                 120                 125
   Ser Gly Gly Thr Ala Ala Leu Gly Cys Leu Val Lys Asp Tyr Phe Pro
       130                 135                 140
   Glu Pro Val Thr Val Ser Trp Asn Ser Gly Ala Leu Thr Ser Gly Val
   145                 150                 155                 160
   His Thr Phe Pro Ala Val Leu Gln Ser Ser Gly Leu Tyr Ser Leu Ser
                   165                 170                 175
   Ser Val Val Thr Val Pro Ser Ser Ser Leu Gly Thr Gln Thr Tyr Ile
               180                 185                 190
   Cys Asn Val Asn His Lys Pro Ser Asn Thr Lys Val Asp Lys Lys Val
           195                 200                 205
   Glu Pro Lys Ser Cys
       210
<210> 13
   <211> 453
   <212> PRT
   <213> Artificial
<220>
   <223> XMab1 -<VEGF> heavy chain
<400> 13
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Thr Phe Thr Asn Tyr
   20 25 30
   Gly Met Asn Trp Val Arg Gln Ala Pro Gly Lys Gly Leu Glu Trp Val
   35 40 45
   Gly Trp Ile Asn Thr Tyr Thr Gly Glu Pro Thr Tyr Ala Ala Asp Phe
   50 55 60
   Lys Arg Arg Phe Thr Phe Ser Leu Asp Thr Ser Lys Ser Thr Ala Tyr
   65 70 75 80
   Leu Gln Met Asn Ser Leu Arg Ala Glu Asp Thr Ala Val Tyr Tyr Cys
   85 90 95
   Ala Lys Tyr Pro His Tyr Tyr Gly Ser Ser His Trp Tyr Phe Asp Val
   100 105 110
   Trp Gly Gln Gly Thr Leu Val Thr Val Ser Ser Ala Ser Thr Lys Gly
   115 120 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Ile Cys Asn Val
   195 200 205
   Asn His Lys Pro Ser Asn Thr Lys Val Asp Lys Lys Val Glu Pro Lys
   210 215 220
   Ser Cys Asp Lys Thr His Thr Cys Pro Pro Cys Pro Ala Pro Glu Leu
   225 230 235 240
   Leu Gly Gly Pro Ser Val Phe Leu Phe Pro Pro Lys Pro Lys Asp Thr
   245 250 255
   Leu Met Ile Ser Arg Thr Pro Glu Val Thr Cys Val Val Val Asp Val
   260 265 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Cys Arg Asp Glu Leu Thr Lys Asn Gln
           355                 360                 365
   Val Ser Leu Trp Cys Leu Val Lys Gly Phe Tyr Pro Ser Asp Ile Ala
       370                 375                 380
   Val Glu Trp Glu Ser Asn Gly Gln Pro Glu Asn Asn Tyr Lys Thr Thr
   385                 390                 395                 400
   Pro Pro Val Leu Asp Ser Asp Gly Ser Phe Phe Leu Tyr Ser Lys Leu
                   405                 410                 415
   Thr Val Asp Lys Ser Arg Trp Gln Gln Gly Asn Val Phe Ser Cys Ser
               420                 425                 430
   Val Met His Glu Ala Leu His Asn His Tyr Thr Gln Lys Ser Leu Ser
           435                 440                 445
   Leu Ser Pro Gly Lys
       450
<210> 14
   <211> 463
   <212> PRT
   <213> Artificial
<220>
   <223> XMab1 -<ANG2> heavy chain
<400> 14
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
                   20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser Ala Ser Val Ala Ala Pro Ser Val Phe Ile Phe Pro Pro Ser Asp
       130                 135                 140
   Glu Gln Leu Lys Ser Gly Thr Ala Ser Val Val Cys Leu Leu Asn Asn
   145                 150                 155                 160
   Phe Tyr Pro Arg Glu Ala Lys Val Gln Trp Lys Val Asp Asn Ala Leu
                   165                 170                 175
   Gln Ser Gly Asn Ser Gln Glu Ser Val Thr Glu Gln Asp Ser Lys Asp
               180                 185                 190
   Ser Thr Tyr Ser Leu Ser Ser Thr Leu Thr Leu Ser Lys Ala Asp Tyr
           195                 200                 205
   Glu Lys His Lys Val Tyr Ala Cys Glu Val Thr His Gln Gly Leu Ser
       210                 215                 220
   Ser Pro Val Thr Lys Ser Phe Asn Arg Gly Glu Cys Asp Lys Thr His
   225                 230                 235                 240
   Thr Cys Pro Pro Cys Pro Ala Pro Glu Leu Leu Gly Gly Pro Ser Val
                   245                 250                 255
   Phe Leu Phe Pro Pro Lys Pro Lys Asp Thr Leu Met Ile Ser Arg Thr
               260                 265                 270
   Pro Glu Val Thr Cys Val Val Val Asp Val Ser His Glu Asp Pro Glu
           275                 280                 285
   Val Lys Phe Asn Trp Tyr Val Asp Gly Val Glu Val His Asn Ala Lys
       290                 295                 300
   Thr Lys Pro Arg Glu Glu Gln Tyr Asn Ser Thr Tyr Arg Val Val Ser
   305                 310                 315                 320
   Val Leu Thr Val Leu His Gln Asp Trp Leu Asn Gly Lys Glu Tyr Lys
                   325                 330                 335
   Cys Lys Val Ser Asn Lys Ala Leu Pro Ala Pro Ile Glu Lys Thr Ile
               340                 345                 350
   Ser Lys Ala Lys Gly Gln Pro Arg Glu Pro Gln Val Cys Thr Leu Pro
           355                 360                 365
   Pro Ser Arg Asp Glu Leu Thr Lys Asn Gln Val Ser Leu Ser Cys Ala
       370                 375                 380
   Val Lys Gly Phe Tyr Pro Ser Asp Ile Ala Val Glu Trp Glu Ser Asn
   385                 390                 395                 400
   Gly Gln Pro Glu Asn Asn Tyr Lys Thr Thr Pro Pro Val Leu Asp Ser
                   405                 410                 415
   Asp Gly Ser Phe Phe Leu Val Ser Lys Leu Thr Val Asp Lys Ser Arg
               420                 425                 430
   Trp Gln Gln Gly Asn Val Phe Ser Cys Ser Val Met His Glu Ala Leu
           435                 440                 445
   His Asn His Tyr Thr Gln Lys Ser Leu Ser Leu Ser Pro Gly Lys
       450                 455                 460
<210> 15
   <211> 498
   <212> PRT
   <213> Homo sapiens
<400> 15
   Met Trp Gln Ile Val Phe Phe Thr Leu Ser Cys Asp Leu Val Leu Ala
   1               5                   10                  15
   Ala Ala Tyr Asn Asn Phe Arg Lys Ser Met Asp Ser Ile Gly Lys Lys
               20                  25                  30
   Gln Tyr Gln Val Gln His Gly Ser Cys Ser Tyr Thr Phe Leu Leu Pro
           35                  40                  45
   Glu Met Asp Asn Cys Arg Ser Ser Ser Ser Pro Tyr Val Ser Asn Ala
       50                  55                  60
   Val Gln Arg Asp Ala Pro Leu Glu Tyr Asp Asp Ser Val Gln Arg Leu
   65                  70                  75                  80
   Gln Val Leu Glu Asn Ile Met Glu Asn Asn Thr Gln Trp Leu Met Lys
                   85                  90                  95
   Leu Glu Asn Tyr Ile Gln Asp Asn Met Lys Lys Glu Met Val Glu Ile
               100                 105                 110
   Gln Gln Asn Ala Val Gln Asn Gln Thr Ala Val Met Ile Glu Ile Gly
           115                 120                 125
   Thr Asn Leu Leu Asn Gln Thr Ala Glu Gln Thr Arg Lys Leu Thr Asp
       130                 135                 140
   Val Glu Ala Gln Val Leu Asn Gln Thr Thr Arg Leu Glu Leu Gln Leu
   145                 150                 155                 160
   Leu Glu His Ser Leu Ser Thr Asn Lys Leu Glu Lys Gln Ile Leu Asp
                   165                 170                 175
   Gln Thr Ser Glu Ile Asn Lys Leu Gln Asp Lys Asn Ser Phe Leu Glu
               180                 185                 190
   Lys Lys Val Leu Ala Met Glu Asp Lys His Ile Ile Gln Leu Gln Ser
           195                 200                 205
   Ile Lys Glu Glu Lys Asp Gln Leu Gln Val Leu Val Ser Lys Gln Asn
       210                 215                 220
   Ser Ile Ile Glu Glu Leu Glu Lys Lys Ile Val Thr Ala Thr Val Asn
   225                 230                 235                 240
   Asn Ser Val Leu Gln Lys Gln Gln His Asp Leu Met Glu Thr Val Asn
                   245                 250                 255
   Asn Leu Leu Thr Met Met Ser Thr Ser Asn Ser Ala Lys Asp Pro Thr
               260                 265                 270
   Val Ala Lys Glu Glu Gln Ile Ser Phe Arg Asp Cys Ala Glu Val Phe
           275                 280                 285
   Lys Ser Gly His Thr Thr Asn Gly Ile Tyr Thr Leu Thr Phe Pro Asn
   290 295 300
   Ser Thr Glu Glu Ile Lys Ala Tyr Cys Asp Met Glu Ala Gly Gly Gly
   305 310 315 320
   Gly Trp Thr Ile Ile Gln Arg Arg Glu Asp Gly Ser Val Asp Phe Gln
   325 330 335
   Arg Thr Trp Lys Glu Tyr Lys Val Gly Phe Gly Asn Pro Ser Gly Glu
   340 345 350
   Tyr Trp Leu Gly Asn Glu Phe Val Ser Gln Leu Thr Asn Gln Gln Arg
   355 360 365
   Tyr Val Leu Lys Ile His Leu Lys Asp Trp Glu Gly Asn Glu Ala Tyr
   370 375 380
   Ser Leu Tyr Glu His Phe Tyr Leu Ser Ser Glu Glu Leu Asn Tyr Arg
   385 390 395 400
   Ile His Leu Lys Gly Leu Thr Gly Thr Ala Gly Lys Ile Ser Ser Ile
   405 410 415
   Ser Gln Pro Gly Asn Asp Phe Ser Thr Lys Asp Gly Asp Asn Asp Lys
   420 425 430
   Cys Ile Cys Lys Cys Ser Gln Met Leu Thr Gly Gly Trp Trp Phe Asp
   435 440 445
   Ala Cys Gly Pro Ser Asn Leu Asn Gly Met Tyr Tyr Pro Gln Arg Gln
   450 455 460
   Asn Thr Asn Lys Phe Asn Gly Ile Lys Trp Tyr Tyr Trp Lys Gly Ser
   465 470 475 480
   Gly Tyr Ser Leu Lys Ala Thr Thr Met Met Ile Arg Pro Ala Asp Phe
   485 490 495
   Ser Gly
<210> 16
   <211> 191
   <212> PRT
   <213> Homo sapiens
<400> 16
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Asn Pro Cys Gly
       130                 135                 140
   Pro Cys Ser Glu Arg Arg Lys His Leu Phe Val Gln Asp Pro Gln Thr
   145                 150                 155                 160
   Cys Lys Cys Ser Cys Lys Asn Thr Asp Ser Arg Cys Lys Ala Arg Gln
                   165                 170                 175
   Leu Glu Leu Asn Glu Arg Thr Cys Arg Cys Asp Lys Pro Arg Arg
               180                 185                 190
<210> 17
   <211> 117
   <212> PRT
   <213> Artificial
<220>
   <223> variable heavy chain domain VH of <VEGF> B20-4.1
<400> 17
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Ile Asn Gly Ser
               20                  25                  30
   Trp Ile Phe Trp Val Arg Gln Ala Pro Gly Lys Gly Leu Glu Trp Val
           35                  40                  45
   Gly Ala Ile Trp Pro Phe Gly Gly Tyr Thr His Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly His Ser Thr Ser Pro Trp Ala Met Asp Tyr Trp Gly
               100                 105                 110
   Gln Gly Thr Leu Val
           115
<210> 18
   <211> 108
   <212> PRT
   <213> Artificial
<220>
   <223> variable light chain domain VL of <VEGF> B20-4.1
<400> 18
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Val Ile Arg Arg Ser
               20                  25                  30
   Leu Ala Trp Tyr Gln Gln Lys Pro Gly Lys Ala Pro Lys Leu Leu Ile
           35                  40                  45
   Tyr Ala Ala Ser Asn Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Asn Thr Ser Pro Leu
                   85                  90                  95
   Thr Phe Gly Gln Gly Thr Lys Val Glu Ile Lys Arg
                       100                 105